# EUROPEAN PATENT SPECIFICATION

(11) **EP 3 739 589 B1**
(45) Date of publication and mention of the grant of the patent: **21.10.2026**
(21) Application number: 19175253.4
(22) Date of filing: 17.05.2019
(51) Int. Cl.: G16B 15/30, G16B 40/20

(54) **METHOD AND SYSTEM FOR BINDING AFFINITY PREDICTION AND METHOD OF GENERATING A CANDIDATE PROTEIN-BINDING PEPTIDE**
VERFAHREN UND SYSTEM ZUR BINDUNGSAFFINITÄTSVORHERSAGE UND VERFAHREN ZUR ERZEUGUNG EINES KANDIDATENPROTEINBINDENDEN PEPTIDS
PROCÉDÉ ET SYSTÈME DE PRÉDICTION D'AFFINITÉ DE LIAISON ET PROCÉDÉ DE GÉNÉRATION D'UN PEPTIDE DE LIAISON À UNE PROTÉINE CANDIDATE

(43) Date of publication of application: 18.11.2020
(73) Proprietor: NEC OncoImmunity AS, 0379 Oslo (NO)
(72) Inventor: Rose, Chris, 1177 Oslo (NO); Eidsaa, Marius, 0379 Oslo (NO); Stratford, Richard, 0379 Oslo (NO); Clancy, Trevor, 0379 Oslo (NO)
(74) Representative: Gill Jennings & Every LLP

(56) References cited:
- KR-A- 20180 052 959
- THAMMAKORN SAETHANG ET AL: "EpicCapo: epitope prediction using combined information of amino acid pairwise contact potentials and HLA-peptide contact site information", BMC BIOINFORMATICS, BIOMED CENTRAL, LONDON, GB, vol. 13, no. 1, 24 November 2012 (2012-11-24), pages 313, XP021134549, ISSN: 1471-2105, DOI: 10.1186/1471-2105-13-313
- N. JOJIC ET AL: "Learning MHC I--peptide binding", BIOINFORMATICS, vol. 22, no. 14, 15 July 2006 (2006-07-15), pages e227 - e235, XP055125396, ISSN: 1367-4803, DOI: 10.1093/bioinformatics/btl255
- RECHE PEDRO A ET AL: "Prediction of MHC class I binding peptides using profile motifs", HUMAN IMMUNOLOGY, vol. 63, no. 9, 1 September 2002 (2002-09-01), US, pages 701 - 709, XP093144571, ISSN: 0198-8859, Retrieved from the Internet <URL:https://pdf.sciencedirectassets.com/271332/1-s2.0-S0198885900X00936/1-s2.0-S0198885902004329/main.pdf?X-Amz-Security-Token=IQoJb3JpZ2luX2VjECkaCXVzLWVhc3QtMSJHMEUCIAnbH0g5mnn5NoLON9OVCxsrZXE4X0rPHqGhaCTg6vMBAiEAqzd14dLfKqhFtuez9ewLVsXPVoNcJBnQbqtJlXti0PUqsgUIQhAFGgwwNTkwMDM1NDY4NjUiDFC4uO56uL4toyb0E> [retrieved on 20240320], DOI: 10.1016/S0198-8859(02)00432-9

## Description

### BACKGROUND TO THE INVENTION

The binding of biological molecules is of interest across the biomedical sciences including bioinformatics, genomics, proteomics, medicine, and pharmacology. Understanding molecular binding has application in the characterisation of biological processes in healthy and diseased tissues, organs, and subjects; in diagnostic, prognostic, and predictive tasks; and in developing, evaluating, and selecting medicines. Without loss of generality, one example is the role of binding in the identification of immunogenic antigens for vaccine development.

In this scenario, a candidate peptide may be chosen for use in a vaccine based on a binding affinity value of the peptide's binding to a target molecule. A candidate peptide may be chosen from a set of candidates based on the anticipated binding, thus accelerating personalised vaccine development and assuring accuracy and efficiency of the antigen or neoantigen.

The identification of immunogenic antigens from pathogens and tumours has played a central role in vaccine development for decades. Over the last 15-20 years this process has been simplified and enhanced through the adoption of computational approaches that reduce the number of antigens that need to be tested. While the key features that determine immunogenicity are not fully understood, it is known that most immunogenic class I peptides (antigens) are generated in the classical pathway through proteasomal cleavage of its parental polypeptide/protein in the cytosol, subsequently transported into the endoplasmic reticulum by the TAP transporters, before being packaged into empty MHC molecules (major histocompatibility complex; also called human leukocyte antigen [HLA] in humans), and then transported to the cell surface and presented to circulatory CD8+ T-cells.

The ability of a peptide to bind to an MHC molecule represents the most important step in determining immunogenicity, as only MHC-bound peptides can bind and activate circulating T-cells. There are now well-populated publicly available databases such as the Immune Epitope Database and Analysis Resource (IEDB; http://www.iedb.org/ as accessed in June 2017) that provide experimentally validated measurements of binding affinity for the most common MHC alleles, along with standard pre-defined cross-validation data sets that have been used in the scientific literature to benchmark and compare binding affinity predictors. There are also well-populated publicly available databases of the composition of certain classes of biological molecules such as the Immuno Polymorphism Database ImMunoGeneTics HLA database (IPD-IMGT/HLA; https://www.ebi.ac.uk/ipd/imgt/hla/ as accessed in June 2017), which provides DNA sequences for many class I and II HLA alleles. Such databases have been used to train various types of models that attempt to predict binding between *de novo* untested biological molecules. While the sources of measured data are constantly expanding there remains many alleles unrepresented in the data.

Attacks on the peptide-MHC binding problem have been classified into three categories (Luo, et al., 2015): position-specific scoring matrices (PSSMs), machine learning methods, and structural methods. In PSSM approaches, a binding prediction is calculated by combining values taken from one or more matrices defined for each peptide residue position. When larger databases became available, PSSM approaches were generally superseded by machine learning methods, in which potentially complex and arbitrarily flexible functions are fitted to examples from potentially large databases. Structural methods model binding in terms of the three-dimensional structure of the molecules using data from crystal structure databases and approximations to the underlying physics. PSSM methods are based on relatively simple mechanistic models, so may be interpreted, but tend to make poorer predictions compared to machine learning methods. Machine learning methods are generally not based on a mechanistic understanding of binding, so cannot easily be interpreted, but achieve state-of-the-art prediction quality. Structural methods have a clear mechanistic interpretation, but prediction is generally not as fast or accurate as for machine learning methods.

It remains a key challenge of industry to provide high-quality predictions of binding affinity while also having a relatively simple mechanistic interpretation.

The earliest attempts to use statistical and machine learning models to predict binding affinity focused on individual MHC alleles and resulted in what are now called allele-specific models in which only the role of the amino acids in the peptide are considered. The current leading allele-specific methods for MHC class I are perhaps NetMHC 4.0 (Andreatta & Nielsen, 2015) and mhcflurry (https://github.com/hammerlab/mhcflurry accessed July 2017), which are machine learning models that fit arbitrary functions to example data using artificial neural networks and then use those fitted functions to make predictions. Allele-specific methods for MHC class II have also been published.

The subsequent increase in available experimental data for a wider range of common alleles facilitated development of pan-allele and pan-specific models that respectively attempt to predict binding affinity for arbitrary alleles or any of a specified set of alleles, using a single model. Unlike allele-specific models, "pan" models implicitly or explicitly consider the amino acids that form the MHC molecule and the peptide. Pan-allele and pan-specific models generally make poorer predictions of binding or binding affinity than allele-specific models, but are applicable to alleles for which there is insufficient data to train an allele-specific model, and to novel alleles that may arise due to mutation (for example in cancer). The current leading MHC class I pan model is perhaps NetMHCpan 4.0 (Jurtz, et al., 2017) which, like its allele-specific equivalent, is based on an artificial neural network. Pan methods for MHC class II have also been published.

Given sufficiently large training sets, machine learning methods tend to make better binding predictions than PSSM or structural models, but the lack of an interpretable mechanistic model of binding may limit their immediate commercial biomedical application outside of academic research. There is a growing body of literature on the properties that automated prediction systems may be required to demonstrate in addition to making good predictions, such as privacy, transparency, accountability, and fairness (NIPS Symposium Organising Committee, 2016). The legislative landscape is also changing to require such properties of automated systems. For example, with respect to automated decisions that significantly affect the health of natural persons, the European Union (EU) General Data Protection Regulation gives EU citizens the right to obtain human intervention in, and explanations of, those decisions (European Parliament & Council, 2016). Meeting such requirements may be easier when more interpretable models are used.

Given the importance to the biologically sciences of understanding and predicting binding and binding affinity between pairs of biological molecules, particularly in the automated development of immunotherapy, there exists a need in the art for a method that can provide high quality predictions on read, retrieved, data, which is based on a plausible mechanistic model that facilitates human interpretation and intervention, and which can provide estimates of uncertainty on predictions such that downstream consumers of predictions can act on those predictions rationally. Similarly, there exists a need to identify a candidate peptide from a set of peptides which is suitable to bind to a target molecule for use in a vaccine while providing a human-interpretable measure of the prediction and a human-interpretable measure of how that estimation has derived.

KR 20180052959 A discloses methods for predicting binding affinity between MHC and peptides.

The publication by Thammakorn Saethang et al., entitled, "EpicCapo: epitope prediction using combined information of amino acid pairwise contact potentials and HLA-peptide contact site information", BMC Bioinformatics, Biomed Central, London, GB, vol. 13, no. 1, 24, November 2012, page 313, discloses an epitope prediction method. Nanopeptides were encoded numerically using a peptide-encoding scheme.

The publication by N. Jojic et al., entitled, "Learning MHC I-peptide binding", Bioinformatics, vol. 22, no. 11, 15 July 2006, pages e227-e235, discloses a structure bases model for which parameters of MHC I-peptide binding can be estimated from additional sources of data about MHC-peptide binding.

### SUMMARY OF THE INVENTION

In general terms, this disclosure presents a concept of predicting pan-allele binding affinity for MHC classes I and II in terms of pairs of amino acids corresponding to contact points between peptides and MHC molecules. A linear model of contact point amino acid pairs leads to models whose parameters can be interpreted.

According to a first aspect of the present invention there is provided a computer-implemented method of predicting a binding affinity value of a query binder molecule to a query target molecule, as set out in appended claim 1. By proximal to one another we mean sufficiently proximal to one another.

In this way, an prediction of binding affinity can be determined in which the model that is used to perform the prediction can be interpreted. In addition to providing high-quality point estimates of binding affinity or binding, the invention may also provide rigorous uncertainty estimates on those point estimates. Rigorous estimates of uncertainty on predictions may facilitate the rational use of the predictions by downstream consumers and may aid in the interpretation of, or intervention in, automated decisions. For example, a prediction that a pair of molecules do bind but with low probability may be overruled by a sceptical expert, whereas a prediction that a pair of molecules do bind and with high probability may be treated differently by the same expert. Automated downstream consumers of binding predictions may be able to make predictions or decisions that rigorously account for uncertainty in their input.

A predictor according to the invention can provide high quality predictions, which are based on a plausible mechanistic model that facilitates human interpretation and intervention, and which can provide estimates of uncertainty on predictions such that downstream consumers of predictions can act on those predictions rationally.

The measured binding affinity value may for example be exact and determined from lab experiments, an approximation or a value greater than or less than a value determined through experimentation. In certain examples the measured binding affinity value may be censored and censoring information may be provided.

The invention may further provide outputting an estimate of probability of accuracy of the predicted binding affinity value.

Preferably the encoded pair of amino acids is encoded as a vector of data elements. Encoding the data elements in this way facilitates the application of a function of each of the contact point pairs to identify how each contributes to a binding affinity value. More preferably each data element is a value indicating presence of an amino acid pairing at each contact point. Probably the value is a binary value indicating if the amino acid pairing is present at the contact point such that there is only one positive binary value for each contact point in the vector. Alternatively, the data element may be a symbol representing an amino acid pair or a matrix of possible amino acid pairs.

Applying a trained machine learning model or statistical model comprises retrieving a set of model coefficients from a data store. The data store may be remote from or local to a location at which the method is performed and kept secret and encrypted. The coefficients may represent a magnitude and direction of contributions to binding affinity for each possible pairing of amino acids. Preferably the coefficients may represent a deviation from a grand mean binding affinity.

In certain embodiments applying a trained machine learning model or statistical model comprises a linear combination of the retrieved coefficients and the encoded pair of amino acids. Such a linear combination is computationally efficient to enable each prediction to be performed quickly and easily on query data with regularity such that when incorporated into a vaccine development pathway, query molecules can be quickly and easily turned into candidate peptides that will likely bind.

The coefficients may be derived by applying a Bayesian estimation algorithm to the encoded reference binder-target pair and the respective associated measured binding value. Statistical distributions may be parameterised. A Bayesian estimation algorithm provides an accurate prediction with an interpretable probability of the binding affinity being accurate and a clear likelihood value such that users can interpret the likelihood of the binding affinity value being accurate and make an informed decision on usage. Similarly, if the binding affinity has a likelihood value below a threshold it may be rejected for usage.

Each reference binder-target pair may be encoded as a sparse matrix, wherein each row represents a reference binder-target pair and wherein each row is associated with a measured binding value. Such encoding in the training process facilitates computational efficiency and storage of the data in, for example, a compressed sparse row storage structure. When training on the data, the sparse matrix encoding improves space and time complexity.

Each row of the matrix may comprise a series of bits, each bit corresponding to a possible pairing of amino acids for each contact point and indicating the specific amino acids present in the contact point pair. Thus there may be one positive value for each contact point which leads to for example a 441-dimensional binary vector for each binder-target pair. Such encoding significantly reduces storage and computational efficiency for example by reducing the dimensions to reduce running time of model fitting and prediction procedures.

A partition of a row of the matrix may encode an amino acid pair as a feature vector that describes a pairing of an amino acid from the reference binder sequence and an amino acid from the target binder sequence. Thus, all of the training data may be represented by one matrix for effective storage and computation with required data being partitioned in the matrix.

The machine learning or statistical model may be trained by estimating a set of coefficients which fit the encoded reference binder-target pairs and the respective associated measured binding affinity values. Fitting techniques may include maximum likelihood for example or regularisation or hierarchical Bayesian estimation.

The method may further comprise outputting a set of parameters associated with the model such that user may interpret if the model is appropriate using known molecules and a binding affinity value for the known molecules. An output in this way may provide for intervention tasks on the process.

The reference data store may further comprise reference binder-target pairs having an associated indication of binding or not binding and the machine learning model or statistical model may be trained by: associating each reference binder-target pair associated with an indication of binding or not binding with an assumed censored IC₅₀ value. The value may for example be below a threshold. Thus, assumed binding peptides can be used to enhance the accuracy of the model and its associated predictions. The training data may contain examples from assays where binding or non-binding may be inferred but binding affinity cannot be measured.

This example in which training is performed on data from assays that provide binding/non-binding results on very large numbers of distinct MHC-peptide complexes could provide a way to dramatically increase the sample size, which may lead to models that make better predictions. A censoring approach allows one, in principle, to combine such data with conventional binding assay data, to provide predictions not only of binding/non-binding, but binding affinity (an IC₅₀ value).

The machine learning model or statistical model may be trained by: associating each reference binder-target pair that has an indication of binding or not binding with an assumed censored IC₅₀ value; and, calculating, for each reference binder-target pair associated with an assumed censored IC₅₀ value, a contribution to binding by integrating an associated statistical distribution over a set of possible binding affinity values. The calculating may be performed based on candidate values of model parameters proposed during model fitting.

In this way the binding predictor may be trained using training data that may contain reference binder-target pairs where binding affinity is known or assumed to be below or above certain values. In certain examples, models trained using training data for which binding affinity has been measured have been observed to make poorer predictions compared to models trained by supplementing the same data set with an approximately equal number of additional reference binder-target pairs for which only censored binding affinity values are available..

In a further example, the machine learning model or statistical model may be trained by: censoring a subset of the measured binding affinity values; calculating a likely binding affinity value corresponding to a censored binding affinity value by integrating an associated statistical distribution over a set of possible binding affinity values; and, associating each reference binder-target pair associated with the censored measured binding affinity values with a calculated likely binding affinity value.

The query binder molecule may be a peptide and/or the second amino acid sequence may be an MHC protein sequence or an HLA protein sequence. Thus the invention has particular utility in determining immunogenicity.

In certain embodiments the method may further comprise comparing the predicted binding affinity value to a threshold value and concluding a query binder molecule will bind based on the threshold value and/or concluding the query binder molecule may be used with the target and is an appropriate candidate.

The present invention is applicable to both MHC class I molecules and MHC class II molecules.

According to a further aspect of the invention there may be provided a method of generating at least one candidate protein-binding peptide in accordance with appended claim 13.

The amino acid sequence of the protein may be obtained by one of: serological antibody testing, oligonucleotide hybridisation methods, nucleic acid amplification based methods (including but not limited to polymerase chain reaction based methods), automated prediction based on DNA or RNA sequencing, de novo peptide sequencing, Edman sequencing or mass spectrometry.

In some comparative examples, the method may further comprise synthesising the one or more candidate peptides.

Additionally, the method may further comprise encoding the candidate peptide into a corresponding DNA or RNA sequence. Further the method may comprise incorporating the sequence into a genome of a bacterial or viral delivery system to create a vaccine.

Thus, peptide, DNA or RNA based vaccines are more reliably constructed for an individual patient as binding affinity can be effectively predicted and the data interpreted.

According to a further aspect of the invention there may be provided a binding affinity prediction system for predicting a binding affinity of a query binder molecule to a query target molecule in accordance with appended claim 14.

According to an example, there may be provided a computer-implemented method of training a machine learning model for use in predicting a binding affinity value of a query binder molecule to a query target molecule, the method comprising: accessing, with at least one processor, a reference data store of reference binder-target pairs comprising respective paired reference binder sequences and reference target sequences, each reference binder-target pair having an associated measured binding value; and, encoding each reference binder-target pair as a plurality of data elements, each data element of the encoded reference binder-target pair representing which amino acids from the respective paired reference binder sequences and reference target sequences are paired at a respective contact point to form a contact point pair, wherein a contact point pair is a pairing of amino acids from a binder molecule and a target molecule which are proximal to one another to influence binding; training a machine learning model or statistical model on the encoded reference binder-target pairs and the measured binding value associated with each reference binder-target pair. Preferably the method further comprises outputting a set of model coefficients for use in predicting a binding affinity value for a query binder molecule and a query target molecule. Preferably the machine learning or statistical model is a mean binding affinity function that models how each pairing of amino acids contributes to binding affinity. Preferably a statistical model fits the encoded reference binder-target pairs to the associated measured binding affinity value.

According to a further aspect, a computer readable medium is provided in accordance with appended claim 15.

### BRIEF DESCRIPTION OF THE DRAWINGS

Embodiments will now be described in detail, by way of example only, with reference to the accompanying figures, in which:
Figure 1 shows a peptide binding to a target molecule;
Figure 2 shows an embodiment of a building a training data set;
Figure 3 shows an embodiment of a method for predicting binding affinity of a binder to a target;
Figure 4 shows an example of a sparse matrix for implementation in examples to encode contact point pairs for a binder-target pair;
Figures 5A and 5B illustrate conceptual matrix visualisations;
Figure 6 illustrates a schematic of a peptide synthesis system;
Figure 7 illustrates a schematic of a server;
Figure 8 shows scatter and ROC plots for an experiment in which a model was trained on the IEDB 2009 data and tested on the IEDB 2013 data;
Figure 9 shows scatter and ROC plots for five-fold cross-validation experiment using the IEDB 2009 and 2013 data;
Figure 10A shows an array of heat maps in which each heat map presents estimates of the model parameter **β** corresponding to one of 62 contact points;
Figure 10B shows a subset of Figure 10A;
Figure 11 shows estimated binding probability ("p_bind") as a function of predicted binding affinity ("y_hat") and marginal histograms for those quantities; and,
Figure 12 shows results for modelling and predicting binding affinity for variable-length sequences (uncensored and censored data).

### DETAILED DESCRIPTION OF THE INVENTION

Methods according to certain embodiments described herein enable computational predictions of a binding affinity value of a query binder molecule, such as a peptide, to a query target molecule, such as a protein. The predictions have particular utility in the identification of personalised vaccines, i.e. the identification of a candidate peptide from a set of candidates which is able to bind to an MHC major histocompatibility complex (MHC) molecule for cancer immunotherapy.

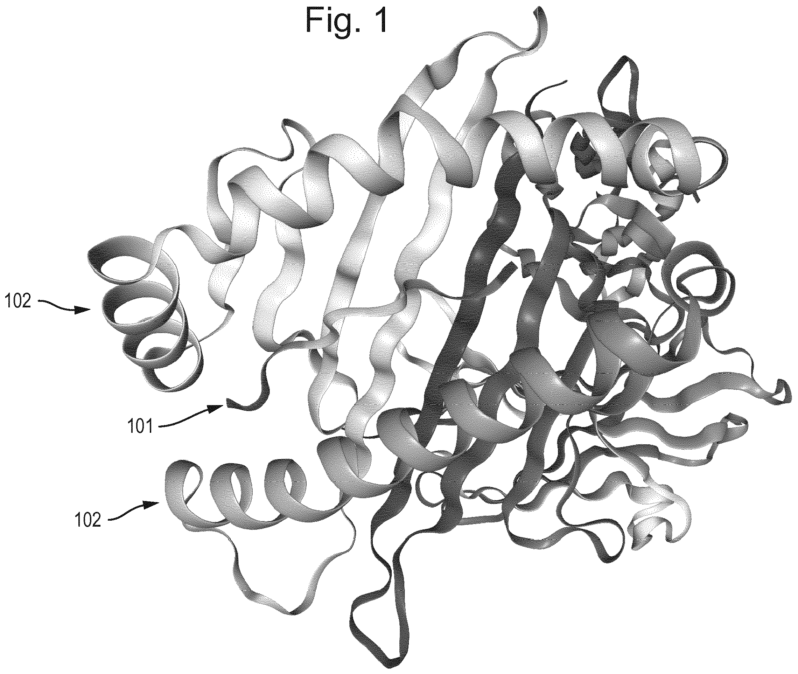

In examples, the binding affinity may be between peptides and MHC molecules. Binding to MHC class I and II molecules is necessary for activation of CD8+ and CD4+ T cells, respectively. This scenario is illustrated by figure 1 which shows a ribbon diagram of nonamer peptide 101 SLYNTIATL bound to an HLA-A*02 MHC class I molecule 102 (1; 2; 3).

While binding affinity can be measured *in vitro* (e.g., using a competition assay), such methods are laborious, expensive, and time-consuming. They cannot feasibly identify all possible antigens from among the plethora of candidates that arise within any given proteome. This issue is particularly acute for the rapid manufacture of vaccines for infectious disease or personalized neoantigen-based cancer vaccines. These scenarios necessitate high-throughput, largely-automated, and reliable predictions, motivating *in silico* approaches.

The query binder molecule and query target molecule of the proposed technique each have a respective amino acid sequence. Predictions are made on the basis of reference data comprising reference binder-target pairs, each pair having a known (measured) binding value, which may be an IC₅₀ value measured in nM, or other value based on IC₅₀, for example. The reference data may also be referred to herein as training data.

The measured binding affinity values need not be a direct measure of binding affinity so long as it reflects the relative binding strength between a binder and a target (i.e., relative to other binder-target pairs). Typically, the reference data may be at least partly obtained from a public database such as the Immune Epitope Database (IEDB) (www.iedb.org), GPCRdb (www.gpcrdb.org) or BRENDA (http://www.brenda-enzymes.org).

In the example reference data, each observation is described by an allele name (MHC class I) or name pair (MHC class II); a species; a peptide sequence; a peptide length; binding affinity between the MHC and peptide molecules expressed as an IC₅₀ value in units of nM; and inequality (censoring) information on the IC₅₀ value.

From this reference data, the technique proposed herein trains a machine learning model which can subsequently be applied to a set of input data, i.e. the query peptide and target module described above in order to identify a candidate peptide for subsequent vaccine synthesis, particularly for cancer immunotherapy.

The proposed technique is based on the principle of considering each specific contact point pair and equating the binding affinity to a sum of the binding contribution of these pairs. A contact point pair may be thought of as a pairing of amino acids from a binder molecule and a target molecule which are proximal to one another to influence binding. Previously proposed techniques do not consider specific pairings of peptide and MHC amino acids. Even if they did the known techniques would be computationally expensive. In the art, in order to accurately train a neural network the known techniques encode each peptide-MHC complex as a pseudosequence, that is, an encoding of the peptide amino acid sequence and a sequence of MHC amino acids believed to be in contact with the peptide.

For background, pairs of molecules are known to bind due to complex dynamic interactions in the electromagnetic field that arise due to the electronic configuration of the molecules. A common model of binding between two biological molecules posits the existence of contact points. Contact points comprise pairs of nucleotides or amino acids in which one member of the pair is from a first molecule and the other member of the pair is from a second molecule. Each pair of nucleotides or amino acids in a contact point are believed to be spatially proximal such that a sufficiently strong electromagnetic force may exist between the nucleotides or amino acids to influence binding between the two molecules. Contact points between two molecules of known sequence can be described by a set of sequence position pairs.

The role of contact points in the peptide-MHC binding problem was considered in the development of NetMHCpan (Nielsen, et al., 2007). Pan-allele models must consider variation between peptides (as for allele-specific models) and polymorphism in the MHC molecule. NetMHCpan encodes peptide and MHC molecule pairs as pseudosequences comprising the peptide amino acid sequence and a sequence of MHC amino acids believed to be in contact with the peptide. Crucially, this encoding does not explicitly model the specific pairings of peptide and MHC amino acids at the contact points, but merely makes relevant variation available to the artificial neural network, which may or may not infer the effect of each specific contact point on binding affinity.

In the literature, molecules are often classified as binding if some measure of binding affinity is less than or greater than a specified value. However, while it is known that even the best binding predictors do not always predict binding correctly, there is no known method that, in addition to providing high-quality point estimates of binding affinity or binding, also provide rigorous uncertainty estimates on those point estimates. Rigorous estimates of uncertainty on predictions may facilitate the rational use of the predictions by downstream consumers and may aid in the interpretation of, or intervention in, automated decisions. For example, a prediction that a pair of molecules bind with low probability may be overruled by a sceptical expert, whereas a prediction that a pair of molecules bind with high probability may be treated differently by the same expert. Automated downstream consumers of binding predictions may be able to make predictions or decisions that rigorously account for uncertainty in their input.

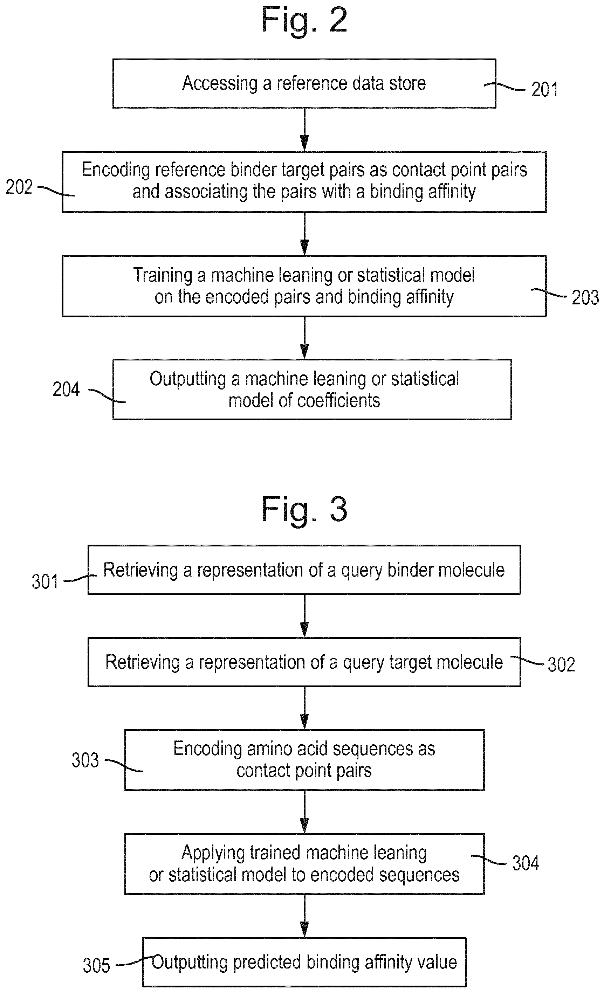

A specific example of the invention will now be described with reference to Figures 2 and 3. The proposed technique may be thought of as two phases. The first is to build a model and the second is to make predictions from that model. A method first comprises a step of accessing a reference data store of reference binder-target pairs (step 201). Each reference binder-target pair comprises a reference binder amino acid sequence, such as a peptide sequence, and a reference target amino and sequence such as a MHC protein sequence. The following discussion will focus on peptide-MHC binding, but it will be understood that the methods and systems discussed below may be readily adapted to other data sets where paired binder sequences and target sequences and corresponding measured binding values are available.

In the reference data, each reference binder-target pair may be associated with a measured binding value. As above, this value may for example be exposed as an IC₅₀ value in units of nM.

However it will be understood that by measured binding value we mean an exact value determined through laboratory experiments, an approximation of the binding value, an indication of binding or not binding or a value greater than or less than a value determined through experimentation. As indicated, binding affinity is typically measured as an IC₅₀ value (in units of nM) using a competition assay in which the concentration of a query peptide is sought that displaces 50% of reference peptides bound to query MHC molecules (or vice versa). IC₅₀ values take a wide range of values and are typically converted to a logarithmic scale for modelling purposes using the transform y = 1 - logb IC₅₀, where b is a sufficiently large logarithmic base (Nielsen, et al., Reliable prediction of T-cell epitopes using neural networks with novel sequence representations, 2003). We model IC₅₀ on this transformed scale and conversion to and from this scale will typically be implicit throughout this disclosure.

In an example, the data store may comprise a representation of peptides deconvoluted from HLA molecules and identified using mass spectrometry, which are confirmed to bind, but when no absolute binding affinity is known.

At step 202, using the reference data store, each reference binder-target pair may be encoded as a set of contact point amino acid pairs, a contact point is a pairing of amino acids from different sequences which are proximal to one another to influence binding. Each encoded pair is associated with a binding affinity value such as a measured binding affinity value or an inequality representative of binding affinity, e.g. <500nM or >500nM.

In effect, this encoding acts as a translation from an amino acid alphabet of 21 symbols (including X as will be described below), to an alphabet of 21 x 21 symbols i.e. 441 symbols. A given symbol describes a peptide-MHC amino acid pair at a contact point, for example the symbol GA would represent a gycine-alanine pair. Each binding affinity value is associated with the encoded contact point pairs.

In an example implementation which will be described in detail below, each reference binder-target pair may be encoded as a matrix of symbols so as to encapsulate the data for further analysis. Preferably this matrix may be a multi-dimensional sparse matrix to facilitate implementation.

From the training data, at step 203, the technique trains a machine learning or statistical model on the encoded pairs and associated binding affinity, from step 202. That is, function is built that models the encoded contact point pairs to the associated binding affinity value. For example, in the specific implementation described below this function may be a linear sum in which the binding affinity value may be calculated as a deviation away from the mean for the encoded matrix (as a row-vector in the specifically described implementation). Thus the function creates a set of model coefficients representing an estimated contribution of each contact point pair to binding affinity. This set of model coefficients is then output at step 204. Again, the binding affinity value may be a measured value or an indication of binding affinity such as an inequality.

Figure 3 illustrates a high level process of how a binding affinity value may be predicted. At step 301, a representation of a query binder molecule is retrieved. At step 302 a representation of a query target molecule is retrieved. As will be understood, the presentation may be an indication of amino acids in sequence in the molecule and may be referred to as amino acid sequences. At step 303, following a similar process used to create the training data from the reference data, the amino acid sequences of the query binder molecule and query target molecule are encoded together as a set of contact point pairs. This may take the form of a vector representing the contact point pairs. In effect this vector is similar to a row-vector of the matrix of encoded reference data.

At step 304, the trained machine learning or statistical model is applied to the encoded contact point pairs. For example, where a linear model is used to create a set of model coefficients, this set of model coefficients is retrieved (not shown) and the encoded contact point pair vector is then multiplied by the model coefficient vector to predict an estimated binding affinity value for the query binder molecule and the query target molecule. The predicted binding affinity value may then be output at step 305. The output may also be, additionally or alternatively, a classification of binding or not-binding, a probability of binding or an indication of binding affinity rather than an exact value.

The output may be utilised in a vaccine development process as discussed elsewhere herein however, the utilisation of the candidate peptide chosen using the technique may be part of a multifactorial choice and may not be chosen solely on the prediction of binding affinity. However in practice the output predicted binding affinity value may be compared to a threshold value and based on the comparison may be considered to bind or not bind. Similarly, the output may choose a peptide or subset of peptides from a set of query peptides based on the best binding affinity predicted.

In practice for example, if the threshold value is 500nM and if the predicted value is above this threshold the query peptide may be said to bind, however a more complex system within which the present techniques may be utilised may incorporate other factors such as processing. 500nM is chosen here as an arbitrary threshold for exemplary purposes. In practice, the threshold may differ for every allele such that 500nM is only a potential threshold.

In the rest of this disclosure, an example implementation of the high level process illustrated in Figures 2 and 3 will be described which provides a more detailed illustration of the encoding process proposed to enable implementation of the technique before a detailed discussion of a technique which has been tested is described together with experimental data demonstrating the efficacy of the concepts described.

A training set is first retrieved which is based on experimental work where, in a lab, someone has performed a competition assay between an MHC molecule, a target/query peptide and a reference peptide. The training set consists of sequences of MHC molecules and sequences of peptides. For each pairing an indication of a binding affinity has been empirically measured. The reason the peptide binds to the MHC molecule is that there is some sort of attraction between them. Work in the field has theorised that the proximity between the peptide amino acid and a molecule amino acid explains this attraction or repulsion. The molecule is based on the principle of contact points, that is, where an amino acid from the peptide is close to the amino acid on the MHC side, so for each contact point we have a pairing of amino acids where one amino acid is from the peptide and one amino acid is from the molecule.

From the training data, a sparse matrix is created to represent each of the contact point pairs for a peptide and MHC molecule pair in the training data. A given row in the matrix may describe the pairings of each amino acid for each contact point. Associated with each row in the matrix is a measured binding affinity value of the training set. In the preferred implementation there exists one matrix for the entire encoded training set, however, this is non-limiting and the symbols may be encoded in other ways for implementation. A single sparse matrix implementation allows for computation efficiency at the encoding and training stage.

Sparsity refers to the idea that many values in the matrix are zero or close to zero. In typical definitions, a matrix of order n is considered to be sparse if it contains much less non-zero element than n². Many alternative definitions of sparse matrix exist. For the purpose of this technique, the fact that the matrix is sparse is not relevant. However the matrix should have a specific encoding to enable the encoding of the contact point pairs and the association of a vector of symbols with an associated measured binding affinity value. This will become evidently clear from the description below. It is a direct result of the method of encoding that the matrix is inevitably sparse and, since it is sparse, this enables the efficient storage of the sparse matrix in well-known sparse matrix storage and computational (i.e. multiplication and sum) techniques.

In other alternatives, for example, a single matrix may be a series of matrices with each matrix representing a contact point pair. However, for brevity will describe only a single matrix representation here to enable the skilled person to understand the principles of the invention.

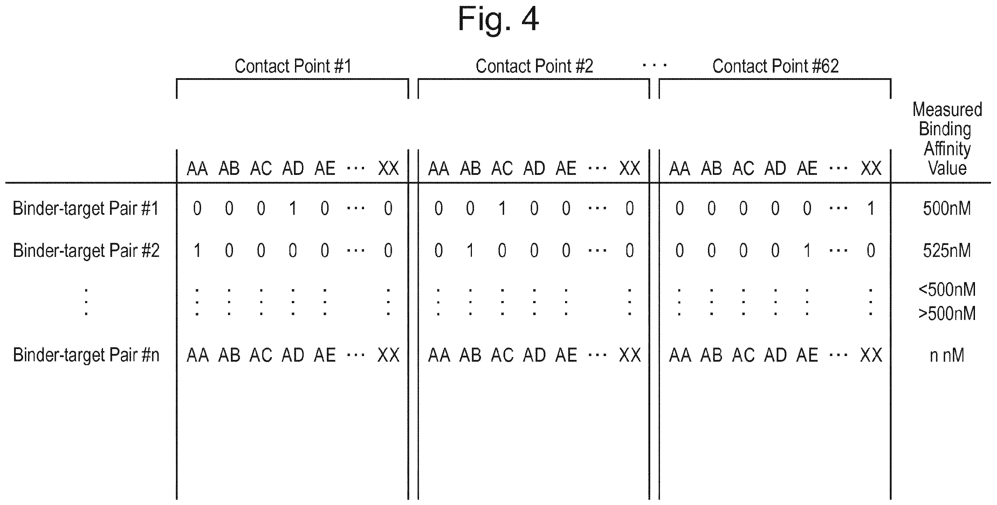

An implementation of a sparse matrix is illustrated in Figure 4 and how a sparse matrix can be designed to implement the principles of the present disclosure. In the sparse matrix example, a row will correspond to a particular MHC molecule and a particular peptide. Each row comprises each contact point and an indication of whether an amino acid is included in each pair for that contact point.

From this encoding method, each row can then be partitioned into its contact points, that is, the row vector can be chopped into smaller row vectors. Partitioning is a way of dividing a matrix in conceptual ways. The matrix can be partitioned into a series of vectors (rows or columns), i.e. a one dimensional list of numbers, or a smaller matrix.

For MHC class I, there are 62 contact points. Thus, each row may be partitioned into 62 vectors, one for each contact point. Each partition is specific to a contact point. Taking the first contact point as an example, this partition represents which amino acid of a peptide is close to which amino acid in the MHC molecule at that contact point. This information needs to be encoded some way in this vector.

20 amino acids are encoded by human DNA. There can therefore be one of 20 amino acids on the MHC molecule side and one of 20 amino acids on the peptide side in each pair. In the exemplary encoding, an X amino acid represents cases where we do not know which amino acid is present or the X may represent an unusual attribute. Thus, in each contact point pair, i.e. contact point partition of the matrix, there is one of 21 amino acids on each side. A pairing is one of 21 x 21 possible pairings. Thus, each contact point partition has 441 possible values.

In the sparse matrix implementation we are describing, only one value is coded. This one value indicates which amino acid from the peptide and which amino acid from the molecule are in proximity with each other.

A column partition of the matrix identifies which contact point the column belongs to and which pairing of peptide amino acid and molecule amino acid may be in proximity. Thus, if it is the value in the column is a '0' this indicates that the amino acids of the column are not in proximity. If the value in the column is a '1' this indicates that the amino acids of the peptide and molecule are in proximity.

While Figure 4 illustrates each contact point as being an encoded number of contact-points using amino acid sequences, there are contemplated myriad alternatives to encoding each contact-point pair. That is, the substance of each position is not essential. By way of example, a reduced amino acid dictionary (or alphabet) may be used for each pair as is known in the art and has been shown to be beneficial in particular scenarios. In another example, each pair may be represented by a binary grouping or by physiochemical properties (e.g. charge) and may be encoded using floating-point numbers (rather than binary encoding) for the representation of each property. Similarly, while we show 62 contact points this number may be varied and each pair may not be represented by all 20 (or 21 including the unknown value described elsewhere) amino acids.

Further, binary representation may be the inverse to that shown where a '0' indicates presence of the pair and '1' indicates absence.

Moreover, the column ordering may be permuted and is shown in this order only for visualisation. Preferably, the dimensionality matches between training and prediction. It is not important in which order the peptide and MHC are sorted. For example, the first "A" may be from the peptide and the second "A" from the MHC molecule but in practice this may be indexed in any order.

It should be emphasised that Figure 4 is not a biological example but merely an example of an encoding.

Figure 4 indicates that each measured binding affinity value associated with each row may be indicated as an exact value or as an inequality such as <500nM or >500nM. This will be explained in more detail elsewhere in this description.

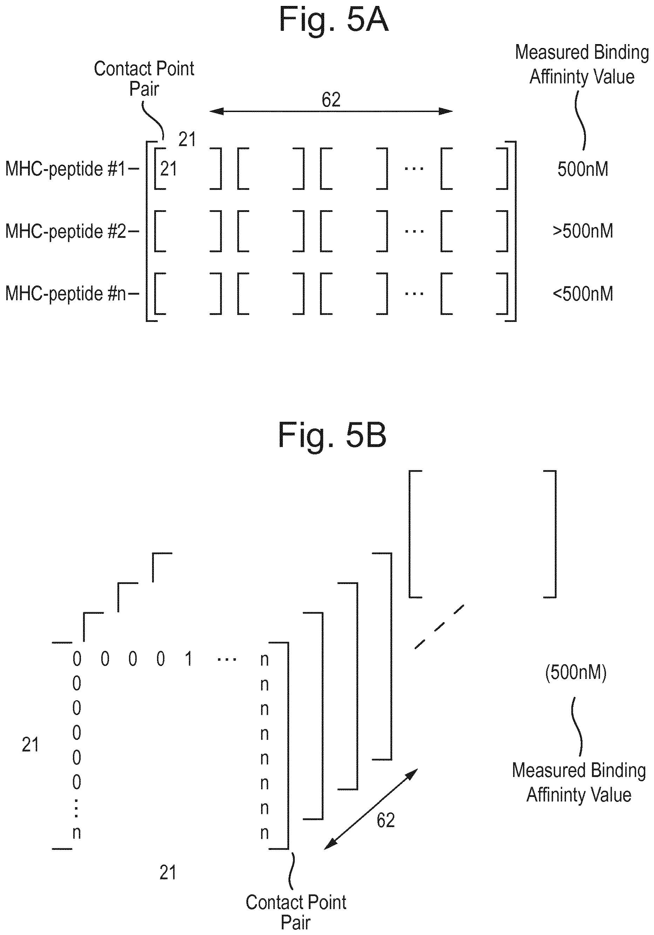

In an alternative implementation illustrated conceptually in Figure 5A, each contact point may be encoded as a matrix such that the matrix is in effect a matrix of embedded matrices. If each element of the matrix is a contact point pair, each column of the matrix would in effect correspond to another matrix. Figure 5B illustrates a further alternative conceptual visualisation of a possible implementation where each contact point is a matrix which combines with the other contact points to create a multi-dimensional matrix representing each peptide-molecule pair.

Returning to Figure 5A to summarise the matrix design, starting from the top left of the matrix, every 441 columns represents a contact point before you move to the next contact point. Each contact point is 21 x 21 items in the row which together form the information which is one contact point. There can only be one long sparse entry for every 441 elements and only 62 non-sparse entries per row. The non-sparse entries show which amino acid pairings are proximal and from this one can derive the amino acid sequences.

The binding affinity value may be associated with each set of pairs separately and may or may not form part of the matrix. Preferably it does not but is associated a data store with each row of the matrix, that is, for every measurement of binding affinity you have one row in the matrix.

In an alternative implementation the matrix may include symbols corresponding to the pair, for example GA, AB, etc. rather than a binary value representing each possible amino acid pairing in the contact point.

Each of the above examples are conceptual visualisations of the techniques proposed. However, what is important is taking the contact point pair and encoding the contribution of these in some form and associating them with the measured binding affinity value.

It should be noted at this stage that while the visualisations of Figures 5A and 5B bear semblance to previous work in the field such as position-specific scoring matrices (PSSM) used to capture motifs in biological sequences, as noted elsewhere in this document, such methodologic do not model interactions in the same way as proposed here. Such methodologies do not consider pairings of contact points (encoding contact point pairs) and most are not pan-allele, that is, the proposed methodologies consider not just individual MHC molecules but allow binding predictions for all MHC molecules.

The technique at this stage has created a data representation from the reference data which may be stored permanently or temporarily in memory.

The next step of the specific implementation is to create the function, in this example a linear sum or linear regression model. In the function, a product of two vectors is performed. The first vector is a row from the matrix and the second is a vector of model coefficients estimated from the training data. A sum of the contributions to binding affinity is just one example of the techniques proposed and is merely an example of a function mapping contact point pairs to binding affinity. Conceptually any function may be provided. For brevity, we have not described that the training data may include censoring information. These examples are given merely to illustrate important concepts.

Thus, from the matrix and the training data, there must be a series of unknown numbers estimated. Using Bayesian estimation which will be discussed below, a set of coefficients can be determined which will lead to an approximation of the binding affinity or as close as possible to it when used as a product of the row vector. As it is an approximation, the exact binding affinity may not need to be known.

Thus, the outcome of the training process is vector of coefficients, preferably stored in a data store.

Once the vector of coefficients is known, the coefficients can be used to predict a binding affinity value for a query binder and target. First, the query peptide and MHC molecule are received. Then, the peptide and MHC molecule are encoded to produce a vector representing the contact point pairs in a similar manner as above. That is a sparse vector where each bit in the vector represents the existence of an amino acid pairing at the contact point. It will be recalled that there will be 62 non-sparse bits in the vector and 440x62 sparse bits. This vector is then multiplied with the coefficient vector to produce a predicted binding affinity value.

As will be understood, this column vector of coefficients need only be built once and in practice the stored column vector values may be utilised for new peptide queries. The column vector may be encrypted or stored for coefficient security and may be kept secret and interpreted using a request-response or query based paradigm.

Since the presence of a pair is indicated by '0' or '1', each coefficient can be thought of as a weight with the binding affinity being a weighted sum of the contact point pairs present in the queried combination. That is, each pair is a '1' which is weighted by the coefficient to derive a value.

For every possible pairing of amino acids from the MHC molecule, the every possible amino acid from the peptide, for every contact point we have a number. That number represents the contribution of binding for each pairing at that contact point. In practice, during a linear model or linear regression, there may be a grand mean. Each coefficient may represent deviation away from the grand mean. Thus, a further column may be introduced in the training matrix representing all '1s'. An additional single element in the coefficient may represent the grand mean. Using a deviation from the mean in this way aids computational efficiency. As will be well-known to the skilled person, other alternatives are possible to address this computational challenge using well-known techniques from the linear regression, linear modelling or other techniques proposed to provide a function between the contact point pair and the binding affinity.

In sum, the present invention can be viewed as a PSSM-like method that incorporates a model of the three-dimensional structure of the molecules, and which is constructed to exploit recent advances in statistical modelling and machine learning: it can make high-quality predictions while also having a relatively simple mechanistic interpretation.

Using the proposed encoding of amino acid pairs at each contact point, any machine learning algorithm may be used. The key idea behind the encoding is to represent the data according to a plausible model of the binding mechanism, in a way that facilitates the application of a statistical model or machine learning method.

However, the encoding results in a fairly high-dimensional sparse design matrix. Some statistical models and machine learning methods have properties that mean they "struggle" with such design matrices. One example is a linear model fitted using the well-known least squares method. The horseshoe estimator adopted is one (Bayesian) way to address that problem. There are others, but the horseshoe has some nice properties.

For example, one alternative to the horseshoe is ridge regression. However, this requires that the researcher specifies the value of a parameter that controls an aspect of the model fitting. It is difficult to reason about that parameter, and in practice it is chosen by trial and error. The horseshoe addresses this problem by linking its version of this parameter to the "noise level" of the quantity being predicted (binding affinity, in this example). The researcher does not need to choose the value of this quantity as the method estimates it from the training data.

In general, model-fitting is typically considered a one-time task and the time it takes to create the coefficients is not important. In practice it is known to fit models using many computing devices running in parallel via cloud computing. However, linear models such as those described for use in the techniques herein (Bayesian or otherwise) are typically very fast at the stage of prediction (which is typically performed many times when used in a product). In context, when the technique is utilised to develop patient-specific immunotherapies the process may be easily replicable to evaluate multiple candidate peptides using the stored coefficients.

In fields where the exact mechanisms underlying a process are not fully understood or where simulating those processes to the required degree of fidelity is intractable (e.g., due to economic, time, or other limitations), statistical and machine learning methods are beneficial, because a useful approximation to the process can be learned using training data (Hastie, Tibshirani, & Friedman, 2009). While there is no hard distinction between machine learning methods and statistical models, machine learning methods are often used to model processes where a fundamental understanding of the mechanisms involved in the process is lacking, while statistical models are often used where an approximation to the mechanisms can be posited and where an interpretation of the model and its predictions is desirable.

Where it is known that certain inputs result in certain outcomes and a mechanism can be posited, a statistical model can often be formulated and values of the model's parameters may be estimated such that the outcomes can be explained under the model and its parameter values in terms of the inputs. In both machine learning methods and statistical models, estimated model parameter values can be used with the model to predict outcomes for *de novo* inputs. The estimated parameter values of a statistical model can often be interpreted with respect to the posited mechanisms to help understand the model and the posited or actual mechanisms. The ability to interpret facilitates development of falsifiable hypotheses that may allow the model to be improved or may have other immediate applications. For example, in the vaccine development context, estimated parameter values may be used to determine how to modify a vaccine to improve its efficacy. In another example, estimated values of model parameters that quantify uncertainty may be used to rationally choose which of a multiplicity of potentially expensive measurements to acquire to improve a training or test set. The ability to intervene facilitates several applications. For example, in the personalised medicine context, a patient with a life-threatening disease may dispute an automated decision that they are unlikely to benefit from a specific therapy. A skilled person could intervene to verify the automated calculation or use their expert knowledge of the disease, therapy, and model to overrule the automated decision.

A statistical model may be fitted to a data set comprising a multiplicity of representative examples of the process to be modelled (this is also known as estimating the model parameters, or training the model). An encoding step is typically required to convert the representative sample into a structured form that is amenable for use within a statistical model. The chosen mathematical forms of the statistical model and encoding usually have a substantial effect on the ability of the model to: be fitted to training data; make predictions for *de novo* examples; be interpreted; and facilitate intervention. The solution described herein provides a particularly effective teaching of which encodings and which statistical models should be used for predicting binding between pairs of biological molecules.

As is described above, encoded nucleotide or amino acid pairs, their corresponding binding affinity values, and corresponding censoring information may be provided as training data to the statistical model. In a particularly preferential implementation, each encoded nucleotide or amino acid pair represents the pair of nucleotides or amino acids at one of a multiplicity of contact points between two molecules, where the first element of a pair comes from a molecule of a first type and the second element of that pair comes from a molecule of a second type. The contact points may originate from studies of the structure of pairs of bound molecules or be inferred using a statistical or machine learning model.

The encoded nucleotide or amino acid pairs may be represented as a design matrix. Each row of the design matrix may represent one example, comprising encoded nucleotide or amino acid pairs for a pair of biological molecules that may bind. The design matrix may be partitioned column-wise such that each partition of a row represents a specific nucleotide or amino acid pair for the example represented by that row. A partition of a given row may encode a nucleotide or amino acid pair as a feature vector that uniquely or non-uniquely describes the pairing of a specific nucleotide or amino acid from the corresponding first molecule with a specific nucleotide or amino acid from the corresponding second molecule. Non-unique encoding permits use of reduced nucleotide or amino acid alphabets (Peterson, Kondev, Theriot, & Phillips, 2009) in which two distinct nucleotides or amino acids are represented by a common symbol from an alphabet. An encoding of a reduced alphabet may be of lower dimensionality than of the full alphabet. As a person skilled in the art will be aware, dimensionality reduction may be advantageous for numerous reasons, including reducing storage requirements and running time of model fitting and prediction procedures.

A preferential encoding uniquely describes a pairing as a binary vector in which all elements of the vector are zero except for a single element whose index indicates the specific nucleotides or amino acids present in the pair (such an encoding is often referred to as "one-hot" or "dummy" encoding). Many other encodings exist, including one-hot encoding with a reference category, BLOSUM encoding (Nielsen, 2003), and VTSA and VHSE encoding (Li, Li, & Shu, 2008), as will be familiar to someone skilled in the art. In a yet more preferential encoding of amino acid pairs, using an alphabet of 20 amino acids (alanine [A], arginine [R], ..., valine [V]) and where the identity of one or both amino acids in each of the pairs may be unknown (usually coded as X), a pair of amino acids may be encoded as a (20+1) × (20+1) = 21 × 21 = 441-dimensional binary vector.

In the preferential case that a binary encoding is used, the design matrix will be sparse. To improve the space and time complexity of the method, the design matrix may be stored in a sparse data structure such as a compressed sparse row (CSR) storage data structure (also known as compressed row storage [CRS]). Other sparse data structures exist, such as compressed sparse column storage (CSC) data structures (also known as compressed column storage [CCS]) and dictionary of keys (DOK), as will be familiar to someone skilled in the art.

Binding affinity values may be represented as a vector where the *i*-th element of the vector gives the binding affinity for the example represented by the *i*-th row of the design matrix. Censoring information may be represented as the sets *L*, *R*, and *U*, whose elements represent indices into the binding affinity vector of left-, right-, and uncensored binding affinities, respectively. However, someone skilled in the art will be aware that there is a multiplicity of ways of representing binding affinity values and censoring information.

Binding affinity measurements are often made using *in vitro* competition assays and are expressed as IC₅₀ values measured in units of nM. IC₅₀ represents the concentration of a molecule of a first type required to displace 50% of a reference molecule bound to a molecule of a second type. Binding affinity values may be transformed using a link function. In a preferential embodiment, the link function is *y* = 1 - log*_{b}* IC₅₀ (Nielsen, 2003), where log*_{b}* is the logarithm with base *b*, and *b* is sufficiently large that an arbitrarily large proportion of binding affinity values in the training set are transformed into the interval [0, 1]. The logarithmic base *b* is preferentially 250,000 nM, however a person skilled in the art will acknowledge that other values may also be suitable. In another preferred embodiment, the link function is *y* = In IC₅₀, where In is the natural logarithm. In yet another preferred embodiment, the link function is the identity function *y* = IC₅₀.

An inverse link function may be defined to compute the binding affinity corresponding to a transformed binding affinity. For example, if the link function is *y* = 1 - log*_{b}* IC₅₀, the inverse link function is IC₅₀ = *b*^{1-*y*}; if the link function is *y* = In IC₅₀, the inverse link function is IC₅₀ = e*^{y}*, where e is Euler's number; and if the link function is the identity function, the inverse link function is also the identity function. The link and inverse link functions may be clamped, such that transformed binding affinities are constrained to the interval [0, 1] and binding affinities are constrained to be greater than zero.

Critically, if the link function is decreasing with respect to IC₅₀ (as in the case of *y* = 1 - log*_{b}* IC₅₀), then each censoring direction must be inverted since, for example, IC₅₀ < 1000 nM implies *y* > 1 - log*_{b}* 1000 nM. Censoring information may be inverted by swapping the indices in the sets *L* and *R*. In the following, the specific link function used (and hence the scale on which IC₅₀ is expressed) and inversion of censoring directions will be implicit unless stated otherwise.

A mean binding affinity function is described that models how an encoded nucleotide or amino acid pair contributes to binding affinity. This function is used in the statistical model along with other information to parameterise statistical distributions, to predict binding affinities for *de novo* pairs of molecules, and in the estimation of probabilities that *de novo* pairs of molecules bind.

The mean binding function may be parameterised by a "grand mean" binding affinity and coefficients that model, for each encoded nucleotide or amino acid pair, the magnitude and direction of a deviation from the grand mean binding affinity associated with the encoded nucleotide or amino acid pair.

The mean binding affinity function is *µ = y̅ +* **x**^{T}**β**, where *y̅* is the grand mean binding affinity, **x**^{T} is a row vector of encoded nucleotide or amino acid pairs for a pair of biological molecules whose binding is of interest (i.e., a row of the design matrix), ^{T} is the transpose operator, **β** is a column vector of coefficients, and **x**^{T}**β** is the dot product of **x**^{T} and **β**. A person skilled in the art will realise that the grand mean term may be incorporated in **x**^{T}**β** via trivial redefinition of **x** and **β**.

The vector **β** may be partitioned in a manner analogous to the partitioning of the columns of the design matrix (and hence **x**^{T}) such that a given partition models the magnitudes and directions of additional contributions to binding affinity for each possible pairing of nucleotides or amino acids for the equivalent partition of **x**^{T}. In a particularly preferential example implementation, the partitions of **x**^{T} and **β** correspond to contact points between a molecule of a first type and a molecule of a second type.

**β** and other parameters **θ** may be estimated by fitting the model to training data. An obvious method for fitting the model to training data is maximum likelihood. However, given that each partition of **x** and **β** may be as large as 441 elements and the number of contact points (partitions) may be approximately 100, **β** may comprise many elements (44,100 in this example). The dimensionality of **θ** may be comparable to that of **β**. If the number of training examples is small relative to the dimensionality of (**β**, **θ**), conventional estimation methods such as maximum likelihood may not be successful. Methods based on explicit or implicit regularisation may be used to estimate **β** and the other parameters **θ**. Regularisation methods can be understood as imposing the assumption that observed data can be well modelled via parameters such as **β** comprising many values whose magnitudes are sufficiently small as to be negligible (i.e., that is **β** is sparse in practical terms). Regularisation methods essentially convert estimation problems that have an intractable multiplicity of solutions into tractable problems whose solutions generalise well to *de novo* examples, and there is now a large body of literature on this topic (Jin, Maaß, & Scherzer, 2017). A person skilled in the art will be aware of numerous regularised estimation methods such as ridge regression, the LASSO, the elastic net, compressed sensing, and matching pursuit algorithms. In a preferred example implementation, **β** and the other parameters **θ** may be estimated via hierarchical Bayesian estimation as described below.

Hierarchical Bayesian estimation of high-dimensional models may be performed using an optimisation method such as limited-memory Broyden-Fletcher-Goldfarb-Shanno (L-BFGS) (Byrd, Hansen, Nocedal, & Singer, 2016) or stochastic gradient ascent (Robbins & Monro, 1951) to compute a maximum *a posterior*i (MAP) point estimate of **β** and the other parameters. Alternatively, approximate samples from the joint posterior distribution of **β**, **θ** may be drawn using automatic differentiation variational inference (ADVI) (Kucukelbir, Tran, Ranganath, Gelman, & Blei, 2017) or a Markov chain Monte Carlo (MCMC) method such as the No-U-Turn (NUTS) sampler (Hoffman & Gelman, 2014).

Each of these methods requires the ability to calculate a posterior likelihood or log-likelihood value (optionally excluding constant terms), given the training data and proposed values of **β**, **θ**. While the following may be formulated in terms of likelihoods or log-likelihoods, a person skilled in the art will acknowledge that, for computational reasons, it may be advantageous to work with probability masses and densities on a logarithmic scale. The posterior log-likelihood of the parameters **β**, **θ** given design matrix **X,** binding affinities **y**, and censoring information *L*, *R*, *U* may be modelled as:$log f \left(β, θ|X,y,L, R, U\right) = log f \left(y|X, L, R, U, β, θ\right) + log f \left(β, θ\right) - log f \left(X,y,L, R, U\right) ,$ where: *f*(**y**|**X**, *L, R, U,* **β**, **θ**) is the likelihood function (the probability mass or density of **y** conditioned on **X,** *L, R, U,* **β**, **θ**); *f*(**β**, **θ**) is a prior probability mass or density function for **β**, **θ**; and *f*(**X**, **y**, *L, R, U)* is the probability mass or density of **X, y,** *L*, *R*, *U*. Since **X, y,** *L*, *R*, *U* are constant for a given training set, the log *f*(**X**, **y**, *L, R, U)* term may be dropped such that log *f*(**β**, **θ**|**X**, **y,** *L, R, U)* may be computed up to constant additive terms.

A likelihood function may be calculated via a probability mass or density function that models the probability of observing *y* given a predicted mean, *µ*. The likelihood function models stochastic variation (e.g., measurement error) in a quantity measured by an assay from a predicted value not subject to the stochastic variation.

Where binding affinity values in the training set are censored (for example, where only an upper or lower bound on a binding affinity is known), a likelihood corresponding to a censored binding affinity may be calculated by integrating its associated statistical distribution over the possible binding affinity values permitted by the censoring. In this way, the binding predictor may be trained using training data that may contain examples where binding affinity is known or assumed to be below or above certain values. Moreover, the training data may contain examples from assays where binding or non-binding can be inferred, but binding affinity cannot be measured. Examples of the training data include mass spectrometry data. In the MHC class I peptide binding example, data from competition assays that allow binding affinity to be measured could be supplemented by data from peptide elution studies where peptides are merely known to bind or not bind. In an example, wherein binding affinity cannot be measured but binding can be assumed to occur, binding peptides may be assumed to have censored IC₅₀ values below 500 nM; alternatively allele-specific censoring values may be used to model the observation that distinct MHC alleles may have different binding characteristics.

The machine learning model or statistical model may be trained by: associating each reference binder-target pair that has an indication of binding or not binding with an assumed censored IC50 value; and, given candidate values of the model parameters proposed during model-fitting, calculating for each such pair a contribution to the likelihood by integrating an associated statistical distribution over a set of possible binding affinity values.

The log-likelihood function log *f*(**y**|**X**, *L*, *R*, *U*, **β**, **θ**) may therefore be modelled as: *Σ*_{*i*∈*U*} log *f*(*yᵢ*|*y̅* + ***x**ᵢ*^{T}**β**, **θ***ᵢ*) + Σ_{*i*∈*L*} log *F*(*yᵢ*|*y̅ +* **x***ᵢ*^{T}**β**, **θ***ᵢ*) + ∑_{*i*∈*R*} log [1-*F*(*yᵢ*|*y̅ +* **x***ᵢ*^{T}**β**, **θ***ᵢ*)], where *yᵢ* is the i-th binding affinity, **x***ᵢ*^{T} is the i-th row of the design matrix **X, θ*ᵢ*** are parameters for the *i*-th training example of the probability mass or density function *f* and its corresponding cumulative probability mass or density function *F*.

There are numerous probability mass or density functions that may be chosen for *f*, as will be known to someone familiar in the art. In preferred embodiments, *f* is the density function for a normal distribution and the link function is *y* = 1 - log*_{b}* IC₅₀; or *f* is the probability mass function for a Poisson distribution and the link function is *y* = In IC₅₀; or *f* is the probability mass function for a negative binomial distribution and the link function is *y* = In IC₅₀. Depending on the support of the function chosen for *f*, the domain of *y* may be adjusted, for example by rounding real values of *y* to integers.

Computation of the *y̅* + **x***ᵢ*^{T}**β** for all *i* (the indices of the rows of **X** and the elements of **y**) may be performed via a matrix-vector product **Xβ**, and that product may be computed efficiently using sparse linear algebra routines, as will be known to someone skilled in the art.

The posterior log-likelihood may be specified via a hierarchy of prior distributions to model uncertainty in the parameters *y̅*, **β** and **θ**. Uncertainty on *y̅* may be modelled as *y̅* ~ N(*m*₁, *s*₁), where mean *m*₁ and standard deviation *s*₂ are predefined constants. In the embodiment that the log-likelihood function is modelled using normal distributions N(*µᵢ*, *σ*) with means *µᵢ = y̅ +* **x***ᵢ*^{T}**β**, and standard deviations σ, and the link function is *y* = 1 - log*_{b}* IC₅₀, the hierarchy
σ² ~ HC(0, *s*₂)
*βᵢ* ~ N(0, *λᵢ*)
*λᵢ* ~ HC(0, *τ*)
*τ* ~ HC(0, *σ*)
where HC denotes a half-Cauchy distribution and *s*₂ is a predefined constant, defines a horseshoe estimator (Carvalho, Polson, & Scott, 2010) for **β**, **θ,** where **θ** is (*σ*, **λ**, *τ*). In a preferred embodiment, *m*₁ = ½, *s*₁ = 1, and *s*₂ = 1.

In the example that the log-likelihood function is modelled using negative binomial distributions NB(*µᵢ*, *ϕ*) with means *µᵢ* = *y̅ +* **x***ᵢ^{T}***β** and variances *µᵢ* + *µᵢ*²/*ϕ*, where the uncertainty on the over-dispersion parameter *ϕ* is modelled as an improper uniform prior on [0, ∞], and the link function is *y* = In IC₅₀, the hierarchy
*βᵢ* ~ N(0, *λᵢ*)
*λᵢ* ~ HC(0, *τ*)
where *τ* is a predefined constant, defines an estimator for **β**, **θ,** where **θ** is (**λ**, *τ*). In a preferred embodiment, *m*₁ = ½, *s*₁ = 5, and *τ* = 5/2.

A person skilled in the art will observe that if the training set is sufficiently large, the exact values of constants such as *m*₁, *s*₁, *s*₂, and *τ* may be relatively unimportant, and that as *ϕ* → ∞, a negative binomial distribution tends towards a Poisson distribution that may be used in place of a negative binomial distribution if over-dispersion is not supported by the training data.

In the following example there is a method for interpreting a fitted model and for intervening in the use of such a model by presenting estimates of the model parameters using an output medium. In accordance with an example of the proposed solution, estimated values of **β** or **θ** may be presented as an array of heat maps on an output medium such as a computer screen. In such a presentation, each heat map corresponds to a partition of **β** (i.e., a contact point) and within each heat map, the rows may correspond to nucleotides or amino acids from molecules of a first kind and the columns to nucleotides or amino acids from molecules of a second kind, and the hue or intensity of each element of the heat map may correspond to the estimated value of the contribution made by the corresponding pairing of nucleotides or amino acids at the corresponding contact point. Such a presentation may enable a suitably qualified individual to perform an intervention task such as predicting binding affinity for a pair of molecules of known sequence, given the contact point indices used in fitting the model, and the estimated grand mean binding affinity. A person skilled in the art will acknowledge that there are a multitude of ways of presenting such information (e.g., as tables or nomograms) and that there are a multitude of output media (e.g., paper printouts or computerised user interfaces).

A method is provided for predicting binding affinity for *de novo* pairs of molecules using the mean binding affinity function and an estimate of the model's joint posterior parameters. Binding affinity can be predicted for *de novo* pairs of molecules by forming a design matrix in the same way as for the training data. Measured or assumed binding affinity values and censoring information are not required for *de novo* prediction. An estimate of the model's joint posterior parameters, may be a maximum *a posteriori* (MAP) point estimate, a sample from the joint posterior distribution of the statistical model's parameters, or a summary statistic computed from such a sample. In a preferred example, the summary statistic is the mean of the samples from the joint posterior distribution. Given estimated parameters **β**, the binding affinities for the molecules represented by the design matrix **X** may be computed using the mean binding affinity function as *y̅* + **Xβ**.

A method is provided for quantifying the uncertainty on predicted binding affinity for each *de novo* pair of molecules by computing an estimate of the probability that the pair of molecules bind. In one example, this may be estimated by summarising a multiplicity of binding affinity predictions, where each prediction may be made using an estimate of the statistical model's parameters taken from a sample from the joint posterior distribution of the model's parameters. The summary may be the proportion of the multiplicity of predictions that meet a criterion such as a prediction being less than a specified value. In the MHC class I peptide binding example, this proportion may be the proportion of the multiplicity of predictions of IC₅₀ that are below 500 nM.

In another example, it is observed that *λᵢ*² estimates the variance on the corresponding *βᵢ*. In the embodiment, the variances of binding affinity measurements for pairs of molecules described by design matrix **X** may be estimated by ***η***² = σ² + **λ^{T}Xλ**. Statistical distributions parameterised by the *ηᵢ* may then be used to model the uncertainty on predicted binding affinities. In one embodiment, the variation in measured binding affinity for the *i*-th pair of molecules may be modelled by the distribution N(*µᵢ*, *ηᵢ*) where *µᵢ* is the mean predicted binding affinity for the *i*-th pair of molecules. The probability that a measured binding affinity for the *i*-th pair of molecules is less than k is therefore approximately *F*(*k*|*µᵢ*, *ηᵢ*), where *F* is the cumulative distribution function for the normal distribution.

In this document we provide a clear use of the method in design of vaccines. However it will be understood that the techniques described herein could equally apply to designing tailored T-cells that recognise the identified targets. Similarly the techniques could also be used to identify neoantigen burden in a tumour and where this is used as a biomarker, i.e. predicting response to therapy.

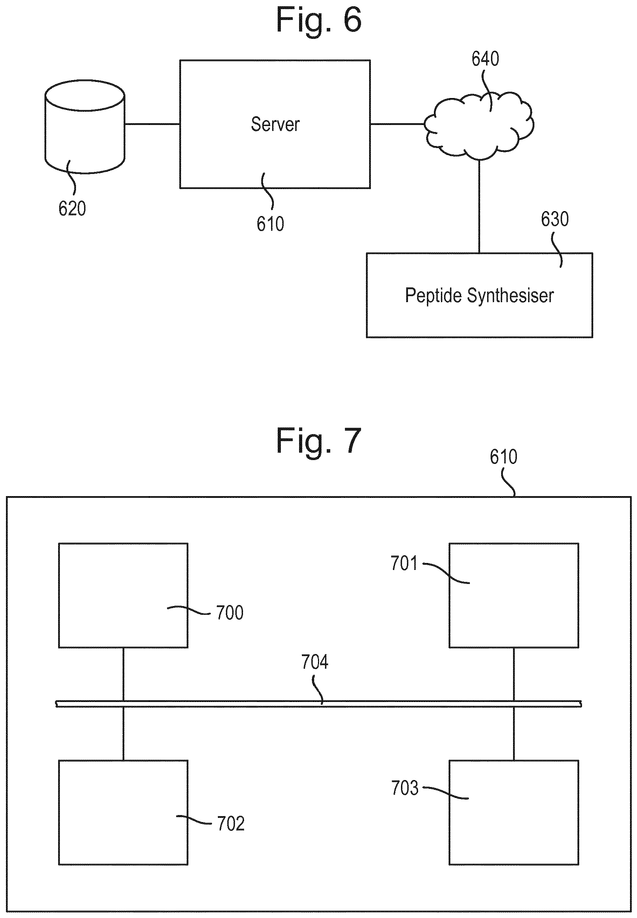

Turning now to Fig. 6, an example of a system suitable for implementing embodiments of the method is shown. The system 600 comprises at least one server 610 which is in communication with a reference data store 620. The server may also be in communication with an automated peptide synthesis device 630, for example over a communications network 640.

In certain embodiments the server may obtain amino acid sequences of a plurality of peptides and an amino acid sequence of a protein and determine, for each peptide, a predicted binding affinity to the protein using the steps described above. Based on the respective predicted binding affinity the server may select one or more candidate peptides of the plurality of peptides.

The candidate peptides may be sent to the automated peptide synthesis device 630 to synthesise the peptide. The automated peptide synthesis device 630 generates target epitopes synthetically, i.e in this example target peptides. Techniques for automated peptide synthesis are well known in the art and it will be understood that any known technique may be used. Typically, the target peptide is synthesized using standard solid phase synthetic peptide chemistry and purified using reverse-phase high performance liquid chromatography before being formulated into an aqueous solution. If used for vaccination, prior to administration the peptide solution is usually admixed with an adjuvant before being administered to the patient. Similarly, the peptide may be encoded in DNA or RNA and used as a vaccine as described elsewhere.

Peptide synthesis technology has existed for more than 20 years but has undergone rapid improvements in recent years. For brevity we do not describe in detail such machines but their operation would be understood to one skilled in the art and such conventional machines may be adapted to receive a candidate protein from the server.

The server may comprise the functions described above to predict binding affinity of a query binder molecule to a query target molecule. The respective binding affinities may be sent to a further processing module to identify a target epitope based on the binding affinity suitable for the creation of a vaccine. However, the server may also be operable to identify a target epitope for vaccine design. It will of course be understood that these functions may be subdivided across different processing entities of a computer network and different processing modules in communication with one another. For example, the server may receive one or more query molecules over a computer network and return a suitable binding affinity or set of candidate epitopes. The query may be received electronical from a computer network or input to a graphical user interface.

The techniques for predicting binding affinity and, based on that binding affinity, for identifying a candidate peptide may integrate into a wider ecosystem for customised vaccine development. Example vaccine development ecosystems are well known in the art and are described at a high-level for context but for brevity we do not describe the ecosystem in detail.

In an example ecosystem, a first, sample, step may be to isolate DNA from a tumor biopsy and matched healthy tissue control. In a second, sequence, step, the data is sequenced and the variants identified i.e. the mutations. In an immune profiler step the associated mutated peptides may be generated «in silico».

Using the associated mutated peptides, and the techniques described here, a neoantigen may be predicted and selected and target epitopes identified for vaccine design. That is, the candidate peptide sequence chosen based on its predicted binding affinity determined using the technique described herein.

The target epitopes are then generated synthetically using conventional techniques as described above. Prior to administration the peptide solution is usually admixed with an adjuvant before being administered to the patient (vaccination).

The suitable target epitopes predicted by the methods described herein may also be used to create other types of vaccine other than peptide based vaccines.

For example, the peptide targets could be encoded into the corresponding DNA or RNA sequence and used to vaccinate the patient, either directly using naked DNA/RNA, or alternatively using a delivery vehicle such as a microparticle, nanoparticle or bacterial delivery system. Note that the DNA is usually inserted in to a plasmid construct. Alternatively the DNA can be incorporated into the genome of a bacterial or viral delivery system (can be RNA also - depending on the viral delivery system) - which can be used to vaccinate the patient - so the manufactured vaccine is a genetically engineered virus or bacteria which manufactures the targets in the patient post immunisation, i.e. in vivo.

An example of a suitable server 610 is shown in Figure 7. In this example, the server includes at least one microprocessor 700, a memory 701, an optional input/output device 702, such as a keyboard and/or display, and an external interface 703, interconnected via a bus 704 as shown. In this example the external interface 703 can be utilised for connecting the server 610 to peripheral devices, such as the communications networks 640, reference data store 620, other storage devices, or the like. Although a single external interface 703 is shown, this is for the purpose of example only, and in practice multiple interfaces using various methods (e.g. Ethernet, serial, USB, wireless or the like) may be provided.

In use, the microprocessor 700 executes instructions in the form of applications software stored in the memory 701 to allow the required processes to be performed, including communicating with the reference data store 620 in order to receive and process input data, and/or with a client device to receive sequence data for query binder molecules and query target molecules, and to generate binding affinity predictions according to the methods described above. The applications software may include one or more software modules, and may be executed in a suitable execution environment, such as an operating system environment, or the like.

Accordingly, it will be appreciated that the server 700 may be formed from any suitable processing system, such as a suitably programmed client device, PC, web server, network server, or the like. In one particular example, the server 610 is a standard processing system such as an Intel Architecture based processing system, which executes software applications stored on non- volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 610 is a shown as a single entity, it will be appreciated that the server 610 can be distributed over a number of geographically separate locations, for example by using processing systems and/or databases that are provided as part of a cloud based environment. Thus, the above described arrangement is not essential and other suitable configurations could be used.

### MATERIALS AND METHODS

### Forming Training Sets

The following sets are a detailed example of an implementation of aspects of the invention together with a set of results derived from this example which demonstrate the utility of the invention in practice.

The datasets BD2009 and BD2013 described by (Kim, et al., 2014) were downloaded from the Immune Epitope Database and Analysis Resource (IEDB) website (http://tools.iedb.org/main/datasets/ accessed August 2016); these datasets will hereafter be referred to as IEDB 2009 and IEDB 2013. A repeatable and uniformly pseudorandom subset of one half of one percent of the IEDB 2009 and 2013 data were obfuscated (weakly encrypted) and set aside for future use. The datasets include examples of: MHC class I alleles names; human or animal species names; peptide sequences; peptide lengths; measured binding affinities between the allele and peptide molecules (expressed as IC₅₀ values in units of nM); and inequality (censoring) information on IC₅₀. Additionally, the datasets specify three different types of five-fold cross-validation partitions (folds), named cv_rnd, cv_sr, and cv_gs. Based on results in (Kim, et al., Dataset size and composition impact the reliability of performance benchmarks for peptide-MHC binding predictions, 2014), the cv_rnd folds were adopted for subsequent experiments.

Release 3.25.0 of the IPD-IMGT/HLA database for DNA sequences of the human MHC was downloaded in extensible mark-up format (XML) from the Anthony Nolan HLA Informatics Group's GitHub repository (https://github.com/ANHIG/IMGTHLA/ accessed August 2016). The XML file was parsed and intermediate data structures formed to represent a mapping from human MHC allele names to quality-controlled amino acid sequences of the α1 and α2 domains of MHC class I alleles translated from the DNA sequences encoding those domains. Similar data structures were constructed to represent mappings from MHC allele names for the animal species present in the IEDB 2009 and 2013 data sets (chimpanzee, gorilla, horse, macaque, and mouse), to quality-controlled amino acid sequences of the α1 and α2 domains of those alleles. Animal amino acid sequences were obtained from sources including the Research Collaboratory for Structural Bioinformatics Protein Data Bank (RCSB PDB, http://www.rcsb.org/pdb/home/home.do) accessed during the second half of 2016.

The IEDB 2009 and 2013 data sets were combined with the MHC class I amino acid sequence data to form a dataset that, in addition to the data in the IEDB 2009 and 2013 data sets, also comprised sequences of the α1 and α2 domains of the MHC class I allele molecule for each peptide. Peptides comprised of nine amino acids (nonamers) are of interest in applications involving MHC class I because the binding groove of the MHC molecule that binds peptides is structured to preferentially bind nonamers. Entries in the combined data set corresponding to peptides of other lengths were removed, leaving entries only for nonamers.

Using data published by (Nielsen, et al., 2007), a data structure was formed that describes 62 pairs of contact point indices into nonamer peptide and α1 and α2 domain amino acid sequences. Each of the 62 pairings represents an amino acid in a nonamer that is believed to be within 4 Å of one of the 182 amino acids of the α1 and α2 domains of an MHC class I molecule to which it may bind, and that hence the two amino acids may interact to affect the binding of the peptide to the MHC molecule. An amino acid alphabet of 21 symbols was used, comprising the 20 standard amino acids encoded by DNA and an X symbol representing an unknown amino acid. The amino acid pairs for the 62 contact points were encoded as a sparse binary design matrix using one-hot encoding and compressed sparse row storage. For computational convenience, censoring information was represented as a vector of indicator values, where the *i*-th indicator value specified the censoring information for the *i*-th binding affinity, with left-censoring encoded as -1, no censoring encoded as 0, and right-censoring encoded as 1. Therefore, the set *L* consists of all indices for which the vector has value -1, the set *R* consists of all indices for which the vector has value 1, and the set *U* consists of all indices for which the vector has value 0. In each of the experiments that follow, binding affinity values were represented as a vector and transformed using a link function as described previously. Transformation of predicted binding affinities back to the IC₅₀ scale using a corresponding inverse link function will be implicit unless stated otherwise. If a decreasing link function (with respect to IC₅₀) was used, the censoring directions were inverted.

The result of these steps was a data set suitable for forming training sets, each comprising a multiplicity of examples of pairs of encoded nucleotide or amino acid sequences, their corresponding binding affinity values, and corresponding censoring information; and for forming corresponding test sets used for validation purposes. The following training and test sets were formed:
i) a training set corresponding to the IEDB 2009 data and a test set corresponding to the IEDB 2013 data;
ii) five-fold cross-validation training and test sets comprising, for each of the five folds defined by the cv_rnd partitioning, a training set comprising all examples except those corresponding to the fold, and a test set comprising all examples corresponding to the fold; and
iii) leave-one-allele-out training sets comprising all data corresponding to the IEDB 2009 and 2013 data except those examples corresponding to each left-out allele, and corresponding test sets comprising data for the left-out alleles.

In each case, the training and tests sets were disjoint such that no model could be evaluated using data on which it had been trained.

### Training On The IEDB 2009 Data And Testing On The IEDB 2013 Data

To estimate how well the proposed method may predict binding affinity and binding for *de novo* pairs of MHC class I and nonamer pairs, a statistical model was fitted to the training set for the IEDB 2009 data (i) described above according to the second and third aspects of the invention. The mean binding affinity function *µ = y̅ +* **x**^{T}**β** was constructed such that partitions of **x** and **β** corresponded to the 62 pairs of contact points between nonamer peptides and the α1 and α2 domains of MHC class I molecules. The log-likelihood function was modelled using normal distributions. The link function *y* = 1 - log*_{b}* IC₅₀ and MAP horseshoe estimation using L-BFGS were used. The resulting model was therefore a pan-allele model of the binding affinity between nonamers and MHC class I molecules.

Binding affinity was predicted for each nonamer-MHC class I molecule pair in the test set for the IEDB 2013 data (i) described above. Quality of binding affinity predictions was evaluated using scatter plots of, and by computing Pearson's correlation coefficient between, the measured and predicted IC₅₀ values on a logarithmic scale. Quality of binding predictions was evaluated by plotting a receiver operating characteristic (ROC) curve and by computing the area under the ROC curve (AUC); true binders were defined as those with measured IC₅₀ values less than 500 nM.

### Five-fold Cross-validation

To estimate the effect of sampling error on summary statistics of prediction quality, five-fold cross-validation was performed using the data sets for the cv_rnd partitioning (ii) described above.

A statistical model was fitted to each left-in fold according to the second and third aspects of the invention. The mean binding affinity function, log-likelihood function, link function, and estimation algorithm were as for the previous experiment.

Binding affinity was predicted for each nonamer-MHC class I molecule pair in each left-out fold. For each left-out fold, binding affinity prediction quality was evaluated using scatter plots of, and computing Pearson's correlation coefficient between, measured and predicted IC₅₀ values on a logarithmic scale. For each left-out fold, binding prediction quality was evaluated by plotting a receiver operating characteristic (ROC) curve and by computing the area under the ROC curve (AUC). True binders were defined as those with measured IC₅₀ values less than 500 nM. The effect of sampling error on correlation coefficients and AUC values was summarised by means and 95% confidence intervals using t distributions.

### Leave-one-allele-out Cross-validation

To estimate the ability of the method to generalise to predict binding affinity for alleles not present in training data, leave-one-allele-out cross-validation was performed using data sets (iii) described above.

A statistical model was fitted to each left-in fold according to the second and third aspects of the invention. The mean binding affinity function, log-likelihood function, link function, and estimation algorithm were as for the previous experiment.

Binding affinity was predicted for each nonamer-MHC class I molecule pair in each left-out fold. For each left-out fold, generalisation was evaluated by computing Pearson's correlation coefficient between the measured and predicted IC₅₀ values on a logarithmic scale; binding prediction quality was evaluated by computing area under the ROC curve (AUC). True binders were defined as those with measured IC₅₀ values less than 500 nM. Results for left-out folds with fewer than 20 IC₅₀ measurements were discarded, since estimates of correlation coefficients and AUC values may be unreliable in such cases. To test if the (human) contact points used in the model allow the model to generalise from human to animal alleles, allele-wise correlation coefficients and AUC values were summarised for each species by means and 95% confidence intervals.

### Interpreting The Model

A statistical model was fitted to the totality of the IEDB 2009 and 2013 data according to the second and third aspects of the invention. The mean binding affinity function, log-likelihood function, link function, and estimation algorithm were as for the previous experiment. According to the fourth aspect of the invention, an array of heat maps was produced to visualise estimated values of **β**. The array was constructed such that each heat map in the array corresponded to one of the contact points defined in (Nielsen, et al., 2007); the rows of a heat map corresponded to peptide amino acids and its columns corresponded to MHC molecule amino acids; and the hue of each element corresponded to the estimated binding affinity contribution at the corresponding contact point.

### Estimating Binding Probabilities

In the example implementation, binding probabilities were estimated for the binding affinity predictions for each nonamer-MHC class I molecule pair in the test set for data set (i) (the IEDB 2013 data). Estimates of the variance of each component of **β** were used, along with σ², to estimate the variance on the predictions, ***η***². A normal distribution, parameterised by the predictions and variances was used to estimate the probability that measured IC₅₀ would be less than 500 nM. These probabilities were plotted as a function of predicted IC₅₀.

### RESULTS

### Training On The IEDB 2009 Data And Testing On The IEDB 2013 Data

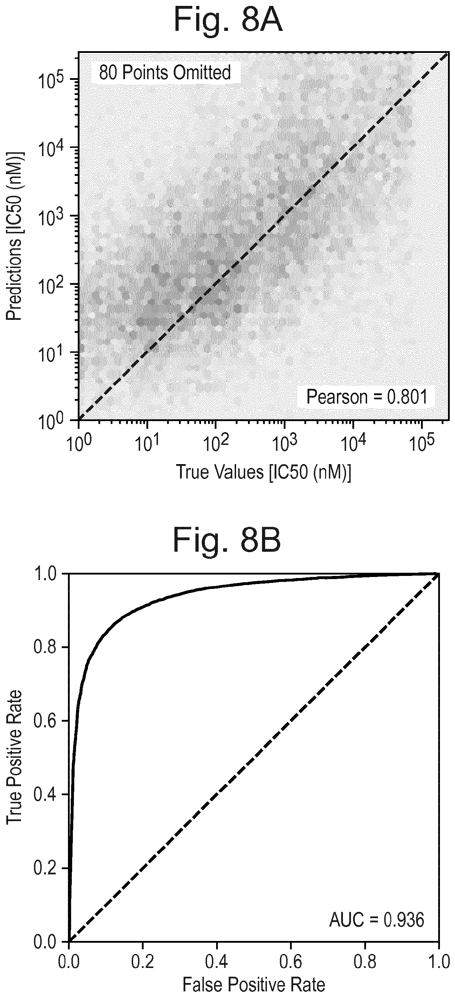

Figure 8 shows scatter and ROC plots for this experiment. Table 1 presents Pearson's correlation coefficient and the area under the ROC curve (AUC) for this experiment.

### Five-fold Cross-validation

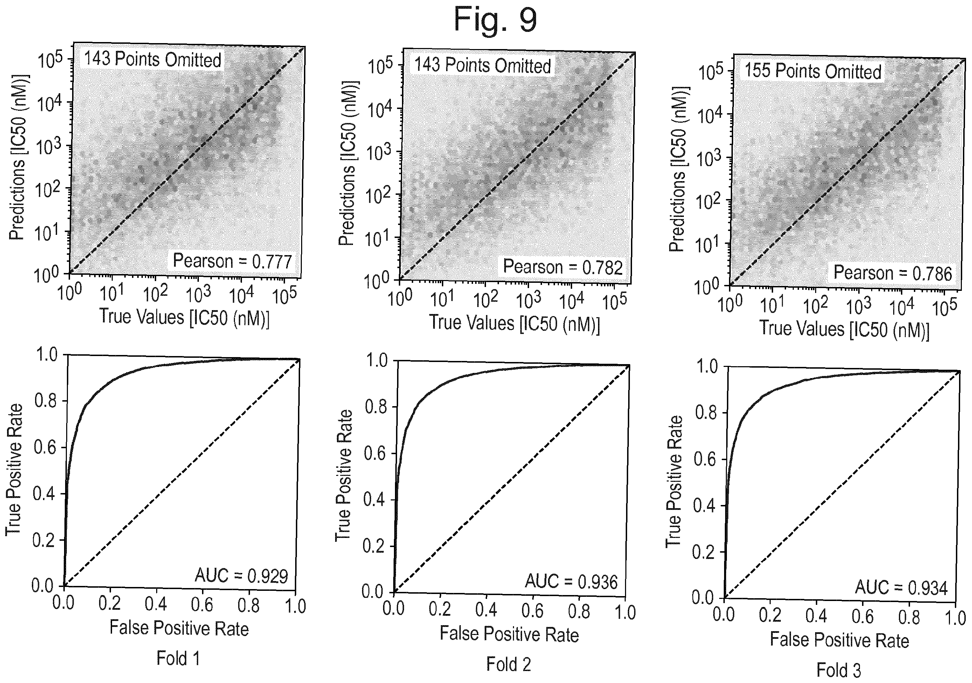

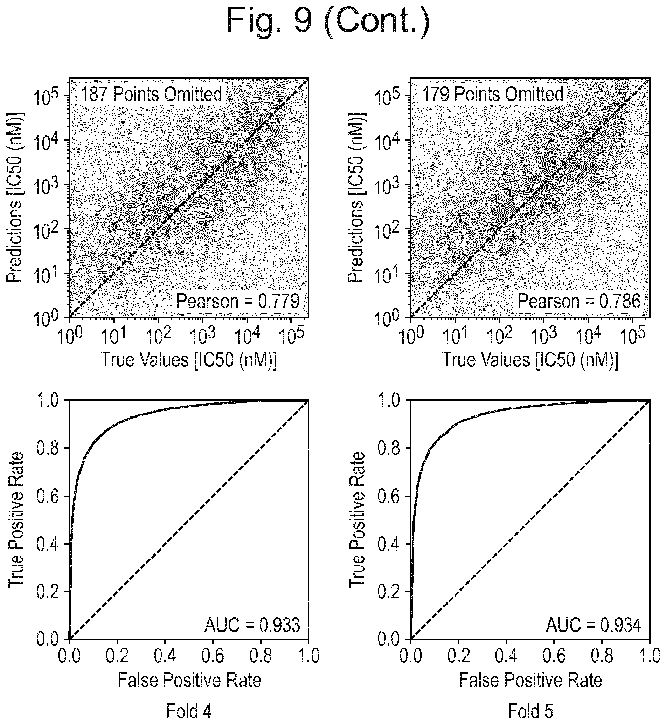

Table 2 presents results for this experiment. Mean Pearson correlation coefficient was 0.782 (95% confidence interval [0.777, 0.787]). Mean AUC was 0.933 (95% confidence interval [0.930, 0.936]). Figure 9 shows the scatter and ROC plots.

### Leave-one-allele-out Cross-validation

Table 3 presents results for this experiment.

### Interpreting The Model

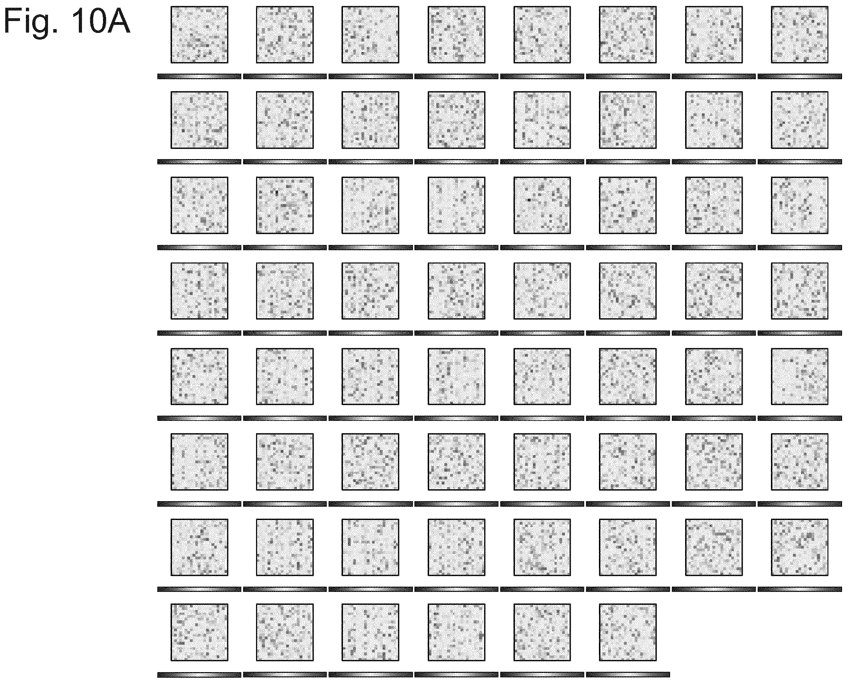

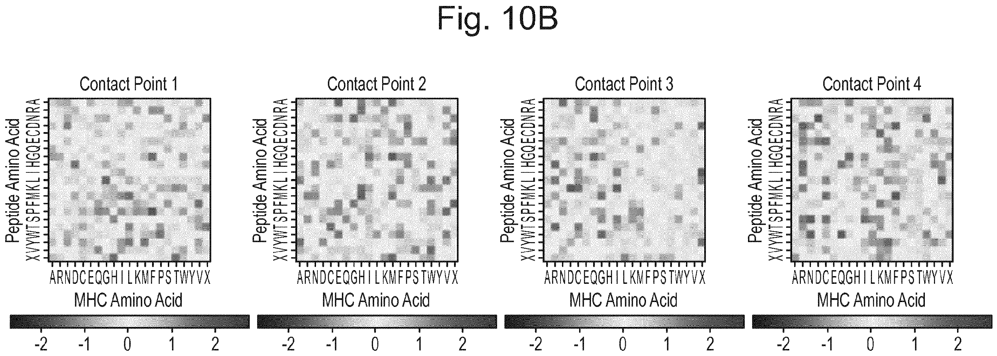

Figure 10A shows an array of heat maps that present estimated parameter values. Figure 10B shows a subset of the array for clarity.

### Estimating Binding Probabilities

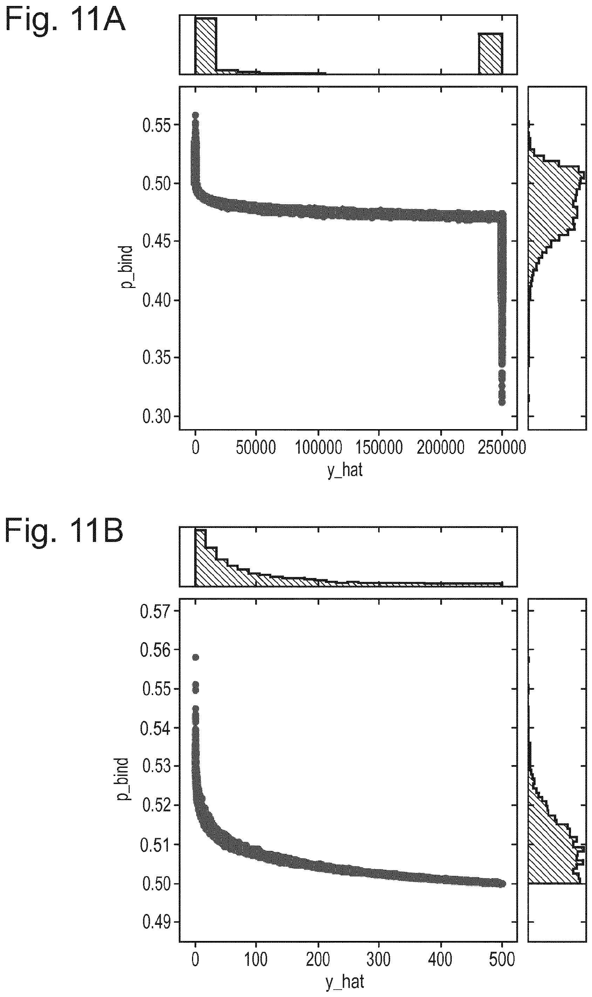

Figure 11 shows plots of estimated binding prediction ("p_bind") as a function of predicted binding affinity ("y_hat"). Marginal histograms of the quantities are also shown. Figure 11a shows that estimated binding probability lies in [0.312, 0.558] over the range of predicted binding affinities [0, 250,000] nM. The sudden decrease in binding probability for predicted binding affinities close to 250,000 nM is due to clipping in the link function. Figure 11b shows the same data for the range of predicted binding affinities [0, 500] nM.

### Training On The IEDB 2009 Data And Testing On The IEDB 2013 Data

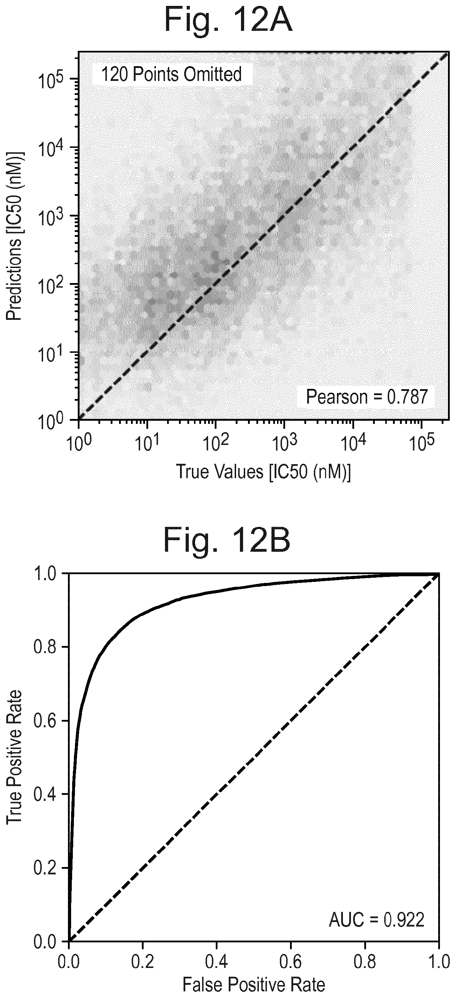

Figure 12 shows a similar image to Figure 8 and shows an evaluation of MHC class I predictions for k-mers, instead of 9-mers.

### DISCUSSION

### Training On The IEDB 2009 Data And Testing On The IEDB 2013 Data

Training on the EIDB 2009 data and testing on the IEDB 2013 data facilitated the calculation of point estimates of Pearson's correlation coefficient and area under the receiver operating characteristic (ROC) curve (AUC), which respectively characterise agreement between measured and predicted IC₅₀ and "true" binders and predicted binding using *de novo* examples that the model was not trained upon. The Pearson correlation coefficient of 0.801 shows that measured and predicted IC₅₀ values are highly but not perfectly correlated.

A binding affinity predictor may be used as a binding predictor (i.e., a classifier) by assigning the label "binder" or "non-binder" to a prediction based on a binding affinity threshold. In the MHC class I peptide binding problem, a threshold of 500 nM is often used, but an arbitrary threshold may be chosen to balance the risk of false positive and negative errors. The AUC value of 0.936 may be interpreted as an estimate of the expected probability that the model would assign a lower predicted binding affinity to a randomly-chosen binding peptide-MHC pair than a randomly-chosen non-binding pair, where the expectation is taken with respect to a uniform distribution of binding affinity thresholds. In practice, a binding predictor based on the method would ordinarily operate using a single pre-specified threshold, so AUC statistics are useful but somewhat artificial. The ROC curve itself facilitates rational threshold choice. The ROC curve shows that, for example, a binding affinity threshold could be chosen such that a binding predictor could operate at a true positive rate of approximately 0.9 if a false positive rate of 0.2 could be tolerated.

### Five-fold Cross-validation

The effect of sampling error on summary statistics of predictions quality was estimated by five-fold cross validation using the cv_rnd partitioning of the IEDB data. Mean Pearson correlation coefficient was estimated to be 0.782 with 95% confidence interval [0.777, 0.787]. Mean AUC was estimated to be 0.933 with 95% confidence interval [0.930, 0.936]. These values are consistent with the point estimates for the IEDB 2013 data in the previous experiment. The shapes of the ROC curves for the five folds are very similar to one another, and also consistent with being able to operate at a true positive rate of approximately 0.9 if a false positive rate of 0.2 could be tolerated.

### Leave-one-allele-out Cross-validation

Leave-one-allele-out cross-validation was performed to estimate the method's ability to generalise to alleles not present in training data. The model demonstrated an ability to generalise to many human alleles. Generalisation to the well-characterised human allele HLA-A-02-01 was excellent (correlation coefficient of 0.830 and AUC of 0.950), and even better to alleles HLA-A-02-199 and HLA-A-02-509 (AUC values of 0.973 and 0.981, for example). However, generalisation to some human alleles, such as HLA-A-01-01, HLA-B-27-03, HLA-B-27-05, and HLA-B-46-01 was poor (AUC values of 0.594, 0.5, and 0.542 respectively).

The model did not generalise as well to animal alleles than it did to human alleles. On average, Pearson's correlation coefficient and AUC were statistically identically to random performance for all mouse alleles except for H-2-Ld. On average, generalisation to human alleles was statistically significantly better than to all other animal species (not correcting for multiple comparisons). This is unsurprising given that the contact points used in the model were determined for human alleles, from which animal alleles are known to differ. That difference would be expected to be greater for species that are evolutionarily more distinct from one another. For example, mouse alleles are known to differ from human alleles in terms of "anchor points" (specific peptide sequence positions that have been found to be particularly important for predicting binding affinity). The anchor point model can be considered as a simplification of the contact point model assumed by the present invention. Generalisation degrades in the studied species as a function of evolutionary distance from humans (mean AUC of 0.830); to chimpanzee (mean AUC of 0.643); to macaque (mean AUC of 0.640); to mouse (mean AUC of 0.575). Poor generalisation to animal alleles compared to human alleles is evidence that modelling binding contributions at contact points is mechanistically plausible.

### Interpreting The Model

Presentation of estimated model parameters may aid a suitably skilled person in interpreting a fitted model. Figures 10A and B show parameter estimates for a model that used a link function that is decreasing with respect to IC₅₀. Positive estimates with large magnitudes correspond to pairings of peptide and MHC amino acids that are associated with small values of IC₅₀ (i.e., stronger binders), and negative estimates with large magnitudes are associated with large values of IC₅₀ (i.e., weaker binders). The figure also illustrates that the estimate of **β** obtained using horseshoe estimation is numerically quite sparse (i.e., many values of the parameter are close to zero), while some have magnitudes that are quite large.

Using such a presentation, it is possible for a suitably skilled person to infer pairings of amino acids that would be expected to be preferentially associated with binding. Hypotheses based on such inferences may then be tested *in silico*, *in vitro*, or *in vivo*.

A suitably skilled person may also intervene in the prediction of a binding affinity given sequences for a peptide and MHC pair, as may be required by law. The person may identify the pair of amino acids corresponding to each contact point, and for each pair read the binding affinity contribution from the corresponding heat map. The sum of these values and the estimated value of the intercept term may then be transformed to the IC₅₀ scale via the appropriate inverse link function, thus providing the binding affinity prediction. The person could verify that automated predictions were computed correctly and experiment with alternative scenarios, for example to investigate how binding affinity may change if the peptide sequence were to be modified.

### Estimating Binding Probabilities

While predicted strong binders have an associated binding probability of little more than 55%, the ability to attach estimates of uncertainty to predictions is nonetheless useful because it may allow downstream consumers of binding predictions to act on those predictions rationally.

### Conclusion

A novel and mechanistically plausible model of binding between pairs of biological molecules has been developed that allows high quality predictions of binding and binding affinity to be made, facilitates human interpretation and intervention, and can provide estimates of uncertainty on those predictions such that downstream consumers of predictions can act on those predictions rationally.

The previously proposed techniques do not consider specific pairings of peptide and MHC amino acids. Even if they did the known techniques would be computationally expensive. To accurately train a neural network the known techniques encode each peptide-MHC complex as a pseudosequence, that is, an encoding of the peptide amino acid sequence and a sequence of MHC amino acids believed to be in contact with the peptide.

The inventive concept is based on the principle of considering each specific contact point pair and equating the binding affinity to a sum of the binding contribution of these pairs.

To encode this, each combination is equivalent to a unique symbol (21² symbols). The pairs are encoded using a sparse matrix with a single non-sparse element representing the specific amino acids present in the pair. To work out how each pair contributes to the binding affinity, and to create training data, the binding affinity is converted to a vector and a deviation from the mean binding affinity is determined (using a dot product of the vectors).

A known Bayesian estimator machine learning technique (probability distribution function, etc.) is used to estimate the deviation from the mean, for a new set of contact point pairs, and then accordingly to determine the most probable binding affinity.

A candidate peptide for use can be chosen from the most probable binding affinity for series of candidate peptides.

A series of examples will now be described in which each example describes certain aspects of the present disclosure.

According to a first example, there may be provided a method for forming a training set comprising a multiplicity of examples of pairs of encoded nucleotide or amino acid sequences, their corresponding binding affinity values, and corresponding censoring information; wherein the nucleotide or amino acid pairs are encoded with one or more encoders; and wherein the number of encoded nucleotide or amino acid pairs in each example, and their interpretations, are invariant across the training set; and wherein the corresponding binding affinity values and censoring information arise from an assay or are assumed based on results from an assay from which binding can be inferred; and wherein for each binding affinity the censoring information specifies that the measured binding affinity value would be expected to be: less than (<) the specified binding affinity or; less than or equal to (≤) the specified binding affinity or; equal to (=) the specified binding affinity or; greater than or equal to (≥) the specified binding affinity or; greater than (>) the specified binding affinity.

In accordance with this example encoded nucleotide or amino acid pairs, their corresponding binding affinity values, and corresponding censoring information may be provided as training data to the statistical model. In a particularly preferential example, each encoded nucleotide or amino acid pair represents the pair of nucleotides or amino acids at one of a multiplicity of contact points between two molecules, where the first element of a pair comes from a molecule of a first type and the second element of that pair comes from a molecule of a second type. The contact points may originate from studies of the structure of pairs of bound molecules or be inferred using a statistical or machine learning model.

The encoded nucleotide or amino acid pairs may be represented as a design matrix. Each row of the design matrix may represent one example, comprising encoded nucleotide or amino acid pairs for a pair of biological molecules that may bind. The design matrix may be partitioned column-wise such that each partition of a row represents a specific nucleotide or amino acid pair for the example represented by that row. A partition of a given row may encode a nucleotide or amino acid pair as a feature vector that uniquely or non-uniquely describes the pairing of a specific nucleotide or amino acid from the corresponding first molecule with a specific nucleotide or amino acid from the corresponding second molecule.

A preferential encoding uniquely describes a pairing as a binary vector in which all elements of the vector are zero except for a single element whose index indicates the specific nucleotides or amino acids present in the pair (such an encoding is often referred to as "one-hot" or "dummy" encoding). In a yet more preferential encoding of amino acid pairs, using an alphabet of 20 amino acids (alanine [A], arginine [R], ..., valine [V]) and where the identity of one or both amino acids in each of the pairs may be unknown (usually coded as X), a pair of amino acids may be encoded as a (20+1) × (20+1) = 21 × 21 = 441-dimensional binary vector.

In the preferential case that a binary encoding is used, the design matrix will be sparse. To improve the space and time complexity of the method, the design matrix may be stored in a sparse data structure such as a compressed sparse row (CSR) storage data structure (also known as compressed row storage [CRS]).

Binding affinity values may be represented as a vector where the i-th element of the vector gives the binding affinity for the example represented by the i-th row of the design matrix. Censoring information may be represented as the sets *L, R,* and *U*, whose elements represent indices into the binding affinity vector of left-, right-, and uncensored binding affinities, respectively.

Binding affinity values may be transformed using a link function. In a preferential embodiment, the link function is *y* = 1 - log*_{b}* IC₅₀ (Nielsen M. L., 2003). The logarithmic base *b* is preferentially 250,000 nM. In another preferred embodiment, the link function is *y* = In IC₅₀, where In is the natural logarithm. In yet another preferred example, the link function is the identity function *y* = IC₅₀.

An inverse link function may be defined to compute the binding affinity corresponding to a transformed binding affinity. For example, if the link function is *y* = 1 - log*_{b}* IC₅₀, the inverse link function is IC₅₀ = *b*^{1*-y*}; if the link function is *y* = In IC₅₀, the inverse link function is IC₅₀ = e*^{y}*, where e is Euler's number; and if the link function is the identity function, the inverse link function is also the identity function. The link and inverse link functions may be clamped, such that transformed binding affinities are constrained to the interval [0, 1] and binding affinities are constrained to be greater than zero.

Critically, if the link function is decreasing with respect to IC₅₀ (as in the case of *y* = 1 - log*_{b}* IC₅₀), then each censoring direction must be inverted since, for example, IC₅₀ < 1000 nM implies *y* > 1 - log*_{b}* 1000 nM. Censoring information may be inverted by swapping the indices in the sets *L* and *R*. In the following, the specific link function used (and hence the scale on which IC₅₀ is expressed) and inversion of censoring directions will be implicit unless stated otherwise.

In a further example there may be provided a mean binding affinity function that models how an encoded nucleotide or amino acid pair contributes to binding affinity. This function is used in the statistical model along with other information to parameterise statistical distributions, to predict binding affinities for *de novo* pairs of molecules, and in the estimation of probabilities that *de novo* pairs of molecules bind.

The mean binding function may be parameterised by a "grand mean" binding affinity and coefficients that model, for each encoded nucleotide or amino acid pair, the magnitude and direction of a deviation from the grand mean binding affinity associated with the encoded nucleotide or amino acid pair.

The mean binding affinity function may for example be *µ* = *y̅ +* **x**^{T}**β**, where *y̅* is the grand mean binding affinity, **x**^{T} is a row vector of encoded nucleotide or amino acid pairs for a pair of biological molecules whose binding is of interest (i.e., a row of the design matrix), ^{T} is the transpose operator, **β** is a column vector of coefficients, and **x**^{T}**β** is the dot product of **x**^{T} and **β**.

The vector **β** may be partitioned in a manner analogous to the partitioning of the columns of the design matrix (and hence **x**^{T}) such that a given partition models the magnitudes and directions of additional contributions to binding affinity for each possible pairing of nucleotides or amino acids for the equivalent partition of **x**^{T}. In a particularly preferential embodiment, the partitions of **x**^{T} and **β** correspond to contact points between a molecule of a first type and a molecule of a second type.

In a further example there may be provided a method for estimating **β** and other parameters **θ** by fitting the model to training data. **β** and the other parameters **θ** may be estimated via hierarchical Bayesian estimation.

Hierarchical Bayesian estimation of high-dimensional models may be performed using an optimisation method such as limited-memory Broyden-Fletcher-Goldfarb-Shanno (L-BFGS) (Byrd, Hansen, Nocedal, & Singer, 2016) or stochastic gradient ascent (Robbins & Monro, 1951) to compute a maximum *a posteriori* (MAP) point estimate of **β** and the other parameters. Alternatively, approximate samples from the joint posterior distribution of **β, θ** may be drawn using automatic differentiation variational inference (ADVI) (Kucukelbir, Tran, Ranganath, Gelman, & Blei, 2017) or a Markov chain Monte Carlo (MCMC) method such as the No-U-Turn (NUTS) sampler (Hoffman & Gelman, 2014).

Each of these methods requires the ability to calculate a posterior likelihood or log-likelihood value (optionally excluding constant terms), given the training data and proposed values of **β, θ**. The posterior log-likelihood of the parameters **β, θ** given design matrix **X,** binding affinities **y,** and censoring information *L, R, U* may be modelled as:$log f \left(β, θ|X,y,L, R, U\right) = log f \left(y|X, L, R, U, β, θ\right) + log f \left(β, θ\right) - log f \left(X,y,L, R, U\right) ,$ where: *f*(**y**|**X**, *L, R, U,* **β, θ**) is the likelihood function (the probability mass or density of **y** conditioned on **X,** *L, R, U,* **β, θ**); *f*(**β**, **θ**) is a prior probability mass or density function for **β, θ**; and *f*(**X**, **y,** *L, R, U*) is the probability mass or density of **X, y,** *L, R, U.* Since **X, y,** *L, R, U* are constant for a given training set, the log *f*(**X**, **y,** *L, R, U*) term may be dropped such that log *f*(**β**, **θ|X, y,** *L, R, U*) may be computed up to constant additive terms.

Where binding affinity values in the training set are censored (i.e., where only an upper or lower bound on a binding affinity is known), a likelihood corresponding to a censored binding affinity may be calculated by integrating its associated statistical distribution over the possible binding affinity values permitted by the censoring. In this way, the binding predictor may be trained using training data that may contain examples where binding affinity is known or assumed to be below or above certain values.

The log-likelihood function log *f*(**y**|**X**, *L, R, U,* **β, θ**) may therefore be modelled as: Σ*_{i∈U}* log *f*(*yᵢ*|*y̅*+ **x***ᵢ*^{T}**β**, **θ***ᵢ* + Σ*_{i∈L}* log *F*(*yᵢ*|*y̅ +* **x***ᵢ*^{T}**β**, **θ***ᵢ*) + Σ_{*i*∈*R*} log [1*-F*(*yᵢ*|*y̅ +* **x***ᵢ*^{T}**β**, **θ***ᵢ*)], where *yᵢ* is the *i*-th binding affinity, **x***ᵢ*^{T} is the *i*-th row of the design matrix **X, θ***ᵢ* are parameters for the *i*-th training example of the probability mass or density function *f* and its corresponding cumulative probability mass or density function *F*.

*f* is the density function for a normal distribution and the link function is *y* = 1 - log*_{b}* IC₅₀; or *f* is the probability mass function for a Poisson distribution and the link function is *y* = In IC₅₀; or *f* is the probability mass function for a negative binomial distribution and the link function is *y* = In IC₅₀.

Computation of the *y̅* + **x***ᵢ*^{T}**β** for all *i* (the indices of the rows of **X** and the elements of **y**) may be performed via a matrix-vector product **Xβ,** and that product may be computed efficiently using sparse linear algebra routines.

The posterior log-likelihood may be specified via a hierarchy of prior distributions to model uncertainty in the parameters *y̅*, **β** and **θ.**

Additionally there may be provided a method for interpreting a fitted model and for intervening in the use of such a model by presenting estimates of the model parameters using an output medium. In accordance with an example embodiment of the proposed solution, estimated values of **β** or **θ** may be presented as an array of heat maps on an output medium such as a computer screen. Such a presentation may enable a suitably qualified individual to perform an intervention task such as predicting binding affinity for a pair of molecules of known sequence, given the contact point indices used in fitting the model, and the estimated grand mean binding affinity.

Further, there may be provided a method for predicting binding affinity for *de novo* pairs of molecules using the mean binding affinity function and an estimate of the model's joint posterior parameters. Binding affinity can be predicted for *de novo* pairs of molecules by forming a design matrix in the same way as for the training data. Measured or assumed binding affinity values and censoring information are not required for *de novo* prediction. An estimate of the model's joint posterior parameters, may be a maximum *a posteriori* (MAP) point estimate, a sample from the joint posterior distribution of the statistical model's parameters, or a summary statistic computed from such a sample. In a preferred embodiment, the summary statistic is the mean of the samples from the joint posterior distribution. Given estimated parameters **β,** the binding affinities for the molecules represented by the design matrix **X** may be computed using the mean binding affinity function as *y̅* + **Xβ.**

There may also be provided a method for quantifying the uncertainty on predicted binding affinity for each *de novo* pair of molecules by computing an estimate of the probability that the pair of molecules bind. In one embodiment, this may be estimated by summarising a multiplicity of binding affinity predictions, where each prediction may be made using an estimate of the statistical model's parameters taken from a sample from the joint posterior distribution of the model's parameters. The summary may be the proportion of the multiplicity of predictions that meet a criterion such as a prediction being less than a specified value. In another embodiment, a normal approximation may be used based on estimates of parameters that model the uncertainty on **β**.

Peptide-MHC binding is central to the study of the adaptive immune system. In vitro binding affinity (IC₅₀) assays cannot scale to large-scale epitope prediction applications (e.g., personalized neoantigen vaccines), motivating accurate in silico approaches. Leading machine learning methods make good predictions but typically lack mechanistic interpretations and do not provide prediction uncertainty estimates. The inventors have developed mechanistic pan-allele models covering MHC classes I and II in which IC₅₀ is predicted as function of amino acid pairs at peptide-MHC contact points. Approximately 40% of IC₅₀ values may be censored in publicly-available binding datasets. The inventors studied bias in common prediction quality metrics such as Pearson's correlation coefficient (PCC) when censored values are treated as measurements and found this practice can overestimate PCC by 12% (simulation) and 18% (experiments on class I data). Excluding censored data to remove such bias from metrics for the models, cross-validated estimates of PCC and area under the receiver operating characteristic curve (AUC) were 0.658±0.01 and 0.834±0.007 (nonamers, class I); 0.668±0.009 and 0.844±0.005 (k-mers, class I); and 0.571±0.02 and 0.779±0.01 (class II). Including censored data, PCC and AUC were estimated to be 0.761±0.009 and 0.923±0.005 (nonamers, class I); 0.755±0.006 and 0.915±0.004 (k-mers, class I); and 0.598±0.02 and 0.793±0.01 (class II). The inventors used rigorous data blinding to test null hypotheses of no overfitting; no such evidence was observed (P>0.05). Models that admit k-mers often attempt to identify nonamer binding cores within longer peptides. Using publicly-available X-ray structure data, it has been demonstrated that the class II model can identify binding cores significantly better than expected by chance (P=0.039). Finally, the inventors have extended the model to infer contact points between nonamer peptides and MHC class I molecules. A class I model trained using inferred contact points performed almost identically to one trained using experimentally-verified contact points, demonstrating it is not necessary to rely on X-ray structure data. This disclosure presented mechanistic models of binding that are competitive with the state-of-the-art, highlighted the importance of treating censored data carefully, and proposed how estimates of prediction uncertainty can be exploited to facilitate rational vaccine design.

### REFERENCES

Byrd, R. H., Hansen, S. L., Nocedal, J., & Singer, Y. (2016). A Stochastic Quasi-Newton Method for Large-Scale Optimization. SIAM Journal on Optimization, 26(2), 1008-1031.
Carvalho, C. M., Polson, N. G., & Scott, J. G. (2010). The horseshoe estimator for sparse signals. Biometrika, 97(2), 465-480.
Hastie, T., Tibshirani, R., & Friedman, J. (2009). The Elements of Statistical Learning: Data Mining, Inference, and Prediction (2nd Edition ed.). Springer. Hoffman, M. D., & Gelman, A. (2014). The No-U-turn sampler: adaptively setting path lengths in Hamiltonian Monte Carlo. Journal of Machine Learning Research, 15(1), 1593-1623.
Jin, B., Maaß, P., & Scherzer, O. (2017, June). Special issue on sparsity regularization in inverse problems. Inverse Problems, 33(6)*.*
Kim, Y., Sidney, J., Buus, S., Sette, A., Nielsen, M., & B., P. (2014). Dataset size and composition impact the reliability of performance benchmarks for peptide-MHC binding predictions. BMC Bioinformatics, 15(214).
Kim, Y., Sidney, J., Buus, S., Sette, A., Nielsen, M., & Peters, B. (2014). Dataset size and composition impact the reliability of performance benchmarks for peptide-MHC binding predictions. BMC Bioinformatics, 15(241).
Kucukelbir, A., Tran, D., Ranganath, R., Gelman, A., & Blei, D. M. (2017). Automatic Differentiation Variational Inference. Journal of Machine Learning Research, 18(14), 1-45.
Li, Z., Li, G., & Shu, M. e. (2008). A novel vector of topological and structural information for amino acids and its QSAR applications for peptides and analogues. Science in China Series B: Chemistry, 51(10), 946-957.
Nielsen, M. L. (2003). Reliable prediction of T-cell epitopes using neural networks with novel sequence representations. Protein Science, 12, 1007-1017.
Nielsen, M., Lundegaard, C., Blicher, T., Lamberth, K., Harndahl, M., Justesen, S., ... Buus, S. (2007). NetMHCpan, a method for quantitative predictions of peptide binding to any HLA-A and-B locus protein of known sequence. PLOS ONE, 2(8), e796.
Peterson, E. L., Kondev, J., Theriot, J. A., & Phillips, R. (2009). Reduced amino acid alphabets exhibit an improved sensitivity and selectivity in fold assignment. Bioinformatics, 25(11), 1356-1362.
Robbins, H., & Monro, S. (1951). A Stochastic Approximation Method. Annals of Mathematical Statistics, 22(3), 400-407.

### TABLES

**Table 1 - Results: Training on IEDB 2009 data and testing on IEDB 2013 data**

| **Pearson's Correlation Coefficient** | **Area Under ROC Curve (AUC)** |
|---|---|
| 0.801 | 0.936 |

**Table 2 - Results: Five-fold cross validation**

| **Fold** | **Pearson's Correlation Coefficient** | **Area Under ROC Curve (AUC)** |
|---|---|---|
| 1 | 0.777 | 0.929 |
| 2 | 0.782 | 0.936 |
| 3 | 0.786 | 0.934 |
| 4 | 0.779 | 0.933 |
| 5 | 0.786 | 0.934 |
| **Mean 95% CI** | **0.782 [0.777, 0.787]** | **0.933 [0.930, 0.936]** |

**Table 3 - Results: Leave-one-allele-out cross-validation**

| **Species** | **Fold** | **Samples** | **Pearson** | **AUC** |
|---|---|---|---|---|
| mouse | H-2-Db | 1790 | -0.1154 | 0.4484 |
| mouse | H-2-Dd | 200 | -0.1197 | 0.3009 |
| mouse | H-2-Kb | 1666 | 0.1090 | 0.5496 |
| mouse | H-2-Kd | 460 | 0.2422 | 0.64 |
| mouse | H-2-Kk | 167 | 0.2924 | 0.6471 |
| mouse | H-2-Ld | 146 | 0.4507 | 0.861 |
| human | HLA-A-01-01 | 3624 | 0.1605 | 0.5937 |
| human | HLA-A-02-01 | 8725 | 0.8296 | 0.9502 |
| human | HLA-A-02-02 | 2424 | 0.7889 | 0.8984 |
| human | HLA-A-02-03 | 4376 | 0.8504 | 0.9444 |
| human | HLA-A-02-05 | 36 | 0.8434 | 0.9419 |
| human | HLA-A-02-06 | 3685 | 0.7319 | 0.873 |
| human | HLA-A-02-07 | 29 | 0.2891 | 0.6818 |
| human | HLA-A-02-119 | 1034 | 0.8562 | 0.9545 |
| human | HLA-A-02-129 | 1141 | 0.5501 | 0.8329 |
| human | HLA-A-02-169 | 889 | 0.5264 | 0.8451 |
| human | HLA-A-02-199 | 1197 | 0.7729 | 0.9725 |
| human | HLA-A-02-509 | 132 | 0.9219 | 0.9814 |
| human | HLA-A-03-01 | 5164 | 0.7268 | 0.9144 |
| human | HLA-A-11-01 | 4387 | 0.7824 | 0.9228 |
| human | HLA-A-23-01 | 1746 | 0.7229 | 0.9012 |
| human | HLA-A-24-02 | 2325 | 0.6960 | 0.8858 |
| human | HLA-A-24-03 | 1159 | 0.7689 | 0.9622 |
| human | HLA-A-25-01 | 926 | 0.6118 | 0.958 |
| human | HLA-A-26-01 | 3510 | 0.6017 | 0.8614 |
| human | HLA-A-26-02 | 614 | 0.5625 | 0.8829 |
| human | HLA-A-26-03 | 513 | 0.6614 | 0.9032 |
| human | HLA-A-29-02 | 1951 | 0.2398 | 0.6232 |
| human | HLA-A-30-01 | 2408 | 0.2871 | 0.656 |
| human | HLA-A-30-02 | 1174 | 0.2899 | 0.6499 |
| human | HLA-A-31-01 | 3809 | 0.7082 | 0.9196 |
| human | HLA-A-32-01 | 827 | 0.4260 | 0.7191 |
| human | HLA-A-33-01 | 1893 | 0.7373 | 0.9056 |
| human | HLA-A-68-01 | 1998 | 0.7413 | 0.8815 |
| human | HLA-A-68-02 | 3625 | 0.6540 | 0.8883 |
| human | HLA-A-69-01 | 2534 | 0.7221 | 0.9107 |
| human | HLA-A-80-01 | 1155 | 0.4261 | 0.8344 |
| human | HLA-B-07-02 | 3595 | 0.6172 | 0.8585 |
| human | HLA-B-08- | 2778 | 0.5405 | 0.8064 |
| | 01 | | | |
| human | HLA-B-08-02 | 991 | 0.5500 | 0.9649 |
| human | HLA-B-08-03 | 450 | 0.6479 | 0.9006 |
| human | HLA-B-15-01 | 3762 | 0.5142 | 0.807 |
| human | HLA-B-15-02 | 161 | 0.2369 | 0.6086 |
| human | HLA-B-15-03 | 578 | 0.6171 | 0.8092 |
| human | HLA-B-15-09 | 807 | 0.5236 | 0.9272 |
| human | HLA-B-15-179-0 | 1420 | 0.4509 | 0.7908 |
| human | HLA-B-18-01 | 2149 | 0.3080 | 0.7442 |
| human | HLA-B-27-03 | 867 | 0.0494 | 0.5 |
| human | HLA-B-27-05 | 2557 | 0.1921 | 0.5531 |
| human | HLA-B-35-01 | 2321 | 0.6494 | 0.8559 |
| human | HLA-B-38-01 | 484 | 0.8952 | 0.9776 |
| human | HLA-B-39-01 | 1516 | 0.6298 | 0.9009 |
| human | HLA-B-40-01 | 2559 | 0.3637 | 0.6742 |
| human | HLA-B-40-02 | 547 | 0.7284 | 0.9097 |
| human | HLA-B-44-02 | 1534 | 0.6798 | 0.9465 |
| human | HLA-B-44-03 | 700 | 0.8202 | 0.9345 |
| human | HLA-B-45-01 | 546 | 0.7117 | 0.8959 |
| human | HLA-B-46-01 | 1781 | 0.0449 | 0.5415 |
| human | HLA-B-48-01 | 858 | 0.6279 | 0.9332 |
| human | HLA-B-51-01 | 1997 | 0.6215 | 0.8925 |
| human | HLA-B-53-01 | 914 | 0.7496 | 0.8922 |
| human | HLA-B-54-01 | 697 | 0.0996 | 0.6024 |
| human | HLA-B-57-01 | 2349 | 0.5198 | 0.8608 |
| human | HLA-B-58-01 | 2700 | 0.5624 | 0.8389 |
| human | HLA-B-58-02 | 30 | 0.2027 | 0.5979 |
| human | HLA-B-73-01 | 114 | 0.2026 | 0.6321 |
| macaque | Mamu-A-01 | 893 | 0.0233 | 0.5015 |
| macaque | Mamu-A-02 | 475 | 0.4441 | 0.7083 |
| macaque | Mamu-A-07 | 32 | 0.4698 | 0.7486 |
| macaque | Mamu-A-11 | 490 | 0.3149 | 0.6555 |
| macaque | Mamu-A-22-01 | 247 | 0.4546 | 0.7952 |
| macaque | Mamu-A-26-01 | 48 | -0.0196 | 0.627 |
| macaque | Mamu-B-01 | 237 | -0.3891 | 0.2813 |
| macaque | Mamu-B-03 | 369 | 0.6534 | 0.8439 |
| macaque | Mamu-B-08 | 366 | 0.3983 | 0.7121 |
| macaque | Mamu-B-17 | 673 | 0.0542 | 0.5219 |
| chimpanzee | Patr-A-01-01 | 203 | 0.1652 | 0.557 |
| chimpanzee | Patr-A-03-01 | 168 | 0.5092 | 0.8226 |
| chimpanzee | Patr-A-04-01 | 143 | 0.3047 | 0.6358 |
| chimpanzee | Patr-A-07-01 | 285 | 0.0822 | 0.5322 |
| chimpanzee | Patr-A-09-01 | 173 | 0.2912 | 0.6083 |
| chimpanzee | Patr-B-01-01 | 450 | 0.0040 | 0.5559 |
| chimpanzee | Patr-B-13-01 | 96 | 0.6887 | 0.8503 |
| chimpanzee | Patr-B-24-01 | 193 | 0.2003 | 0.5797 |
| **mouse** | | 4429 **Mean 95% CI** | 0.1432 [-0.098, 0.384] | 0.575 [0.374, 0.775] |
| **human** | | 110772 **Mean 95% CI** | 0.564 [0.505, 0.623] | 0.830 [0.796, 0.864] |
| **macaque** | | 3830 **Mean 95% CI** | 0.240 [0.016, 0.465] | 0.640 [0.520, 0.759] |
| **chimpanzee** | | 1711 **Mean 95% CI** | 0.281 [0.092, 0.469] | 0.643 [0.539, 0.746] |

## Claims

1. A computer-implemented method of predicting a binding affinity value of a query binder molecule to a query target molecule, the query binder molecule having a first amino acid sequence and the query target molecule having a second amino acid sequence, the method comprising:
encoding (303) the first and second amino acid sequences together as a plurality of data elements to generate an encoded pair of amino acids, each data element of the encoded pair representing which amino acids from the first and second amino acid sequences are paired at a respective contact point between the first amino acid sequence and the second amino acid sequence to form a contact point pair, wherein a contact point pair is a pairing of amino acids from a binder molecule and a target molecule which are proximal to one another to influence binding; and,
applying (304) a trained machine learning or statistical model to the encoded pair of amino acids to predict a binding affinity value, wherein the machine learning model or statistical model is trained by:
accessing, with at least one processor, a reference data store of reference binder-target pairs comprising respective paired reference binder sequences and reference target sequences, each reference binder-target pair having an associated measured binding value; and,
encoding each reference binder-target pair as a plurality of data elements, each data element of the encoded reference binder-target pair representing which amino acids from the respective paired reference binder sequences and reference target sequences are paired at a respective contact point to form a contact point pair,
wherein the model is trained by estimating a set of coefficients which fit the encoded reference binder-target pairs and the respective associated measured binding affinity values; and,
wherein applying a trained machine learning model or statistical model comprises retrieving the set of model coefficients from a data store, and applying a trained machine learning model or statistical model comprises a linear combination of the retrieved coefficients and the encoded pair of amino acids,
such that the predicted binding affinity value is representative of a contribution to binding of each contact point pair of the query binder molecule and the query target molecule.

2. A computer-implemented method according to claim 1, wherein the encoded pair of amino acids is encoded as a vector of data elements.

3. A computer-implemented method according to claim 1 or 2, wherein each data element is a value indicating presence of an amino acid pairing at each contact point.

4. A computer-implemented method according to any preceding claim, wherein the associated measured binding value is censored.

5. A computer-implemented method according to any preceding claim, further comprising outputting an estimate of probability of accuracy of the predicted binding affinity value.

6. A computer-implemented method according to any preceding claim, wherein the coefficients are derived by applying a Bayesian estimation algorithm to the encoded reference binder-target pair and the associated measured binding value.

7. A computer-implemented method according to any preceding claim, wherein each reference binder-target pair is encoded as a sparse matrix, wherein each row represents a reference binder-target pair and wherein each row is associated with a measured binding value.

8. A computer-implemented method according to claim 7, wherein each row of the matrix comprises a series of bits, each bit corresponding to a possible pairing of amino acids for each contact point and indicating the specific amino acids present in the contact point pair and wherein a partition of a row of the matrix encodes an amino acid pair as a feature vector that describes a pairing of an amino acid from the reference binder sequence and an amino acid from the target binder sequence.

9. A computer-implemented method according to any preceding claim, wherein the reference data store further comprises reference binder-target pairs having an associated indication of binding or not binding and wherein the machine learning model or statistical model is trained by:
associating each reference binder-target pair associated with an indication of binding or not binding with an assumed censored IC₅₀ value.

10. A computer-implemented method according to claim 9, further comprising:
calculating, for each reference binder-target pair associated with an assumed censored IC₅₀ value, a contribution to binding by integrating an associated statistical distribution over a set of possible binding affinity values.

11. A computer-implemented method according to any preceding claim, further comprising outputting a set of parameters associated with the model such that user may interpret if the model is appropriate using known molecules and a binding affinity value for the known molecules.

12. A computer-implemented method according to any preceding claim, wherein the query binder molecule is a peptide and/or wherein the second amino acid sequence is an MHC protein sequence or an HLA protein sequence.

13. A method of generating at least one candidate protein-binding peptide, the method comprising:
obtaining amino acid sequences of a plurality of peptides and an amino acid sequence of a protein;
determining, for each peptide, a predicted binding affinity to the protein, by a method according to any one of claims 1 to 12; and
selecting one or more candidate peptides of the plurality of peptides based on the respective predicted binding affinity.

14. A binding affinity prediction system for predicting a binding affinity of a query binder molecule to a query target molecule, the query binder molecule having a first amino acid sequence and the query target molecule having a second amino acid sequence, the system comprising at least one processor in communication with at least one memory device, the at least one memory device having stored thereon instructions for causing the at least one processor to perform a method according to any one of claims 1 to 12.

15. A computer readable medium comprising instructions which, when executed by a processor, cause the processor to perform a method according to any one of claims 1 to 12.

## Patentansprüche

1. Computerimplementiertes Verfahren zur Vorhersage eines Bindungsaffinitätswerts eines Abfrage-Bindermoleküls an ein Abfrage-Zielmolekül, wobei das Abfrage-Bindermolekül eine erste Aminosäuresequenz aufweist und das Abfrage-Zielmolekül eine zweite Aminosäuresequenz aufweist, wobei das Verfahren Folgendes umfasst:
Codieren (303) der ersten und zweiten Aminosäuresequenz zusammen als eine Vielzahl von Datenelementen, um ein codiertes Aminosäurenpaar zu erzeugen, wobei jedes Datenelement des codierten Paars darstellt, welche Aminosäuren von der ersten und zweiten Aminosäuresequenz an einer jeweiligen Kontaktstelle zwischen der ersten Aminosäuresequenz und der zweiten Aminosäuresequenz gepaart werden, um ein Kontaktstellenpaar zu bilden, wobei ein Kontaktstellenpaar eine Paarung von Aminosäuren von einem Bindermolekül und einem Zielmolekül ist, die nah beieinander liegen, um Bindung zu beeinflussen; und,
Anwenden (304) eines trainierten Modells maschinellen Lernens oder eines statistischen Modells auf das codierte Aminosäurenpaar, um einen Bindungsaffinitätswert vorherzusagen, wobei das Modell maschinellen Lernens oder das statistische Modell trainiert wird durch:
Zugreifen mit mindestens einem Prozessor auf einen Referenz-Datenspeicher von Referenz-Binderzielpaaren, der jeweilige gepaarte Referenz-Bindersequenzen und Referenz-Zielsequenzen umfasst, wobei jedes Referenz-Binderzielpaar einen zugeordneten gemessenen Bindungswert aufweist; und,
Codieren von jedem Referenz-Binderzielpaar als eine Vielzahl von Datenelementen, wobei jedes Datenelement des codierten Referenz-Binderzielpaars darstellt, welche Aminosäuren von jeweiligen gepaarten Referenz-Bindersequenzen und Referenz-Zielsequenzen an einer jeweiligen Kontaktstelle gepaart werden, um ein Kontaktstellenpaar zu bilden,
wobei das Modell durch Schätzen eines Satzes von Koeffizienten trainiert wird, die an die codierten Referenz-Binderzielpaare und die jeweiligen zugeordneten gemessenen Bindungsaffinitätswerte angepasst sind; und,
wobei Anwenden eines trainierten Modells maschinellen Lernens oder eines statistischen Modells Abrufen des Satzes von Modellkoeffizienten aus einem Datenspeicher umfasst und Anwenden eines trainierten Modells maschinellen Lernens oder eines statistischen Modells eine lineare Kombination der abgerufenen Koeffizienten und des codierten Aminosäurenpaars umfasst,
sodass der vorhergesagte Bindungsaffinitätswert für einen Beitrag zu Bindung von jedem Kontaktstellenpaar des Abfrage-Bindermoleküls und des Abfrage-Zielmoleküls repräsentativ ist.

2. Computerimplementiertes Verfahren nach Anspruch 1, wobei das codierte Aminosäurenpaar als ein Vektor von Datenelementen codiert ist.

3. Computerimplementiertes Verfahren nach Anspruch 1 oder 2, wobei jedes Datenelement ein Wert ist, der Vorhandensein einer Aminosäurepaarung an jeder Kontaktstelle anzeigt.

4. Computerimplementiertes Verfahren nach einem vorstehenden Anspruch, wobei der zugeordnete gemessene Bindungswert zensiert ist.

5. Computerimplementiertes Verfahren nach einem vorstehenden Anspruch, weiter umfassend Ausgeben einer geschätzten Wahrscheinlichkeit von Genauigkeit des vorhergesagten Bindungsaffinitätswerts.

6. Computerimplementiertes Verfahren nach einem vorstehenden Anspruch, wobei die Koeffizienten abgeleitet werden durch Anwenden eines Bayes-Schätzalgorithmus auf das codierte Referenz-Binderzielpaar und den zugeordneten gemessenen Bindungswert.

7. Computerimplementiertes Verfahren nach einem vorstehenden Anspruch, wobei jedes Referenz-Binderzielpaar als eine dünn besetzte Matrix codiert wird, wobei jede Zeile ein Referenz-Binderzielpaar darstellt, und wobei jede Zeile einem gemessenen Bindungswert zugeordnet ist.

8. Computerimplementiertes Verfahren nach Anspruch 7, wobei jede Zeile der Matrix eine Serie von Bits umfasst, wobei jedes Bit einer möglichen Paarung von Aminosäuren für jede Kontaktstelle entspricht und die spezifischen Aminosäuren anzeigt, die in dem Kontaktstellenpaar vorhanden sind, und
wobei eine Partition einer Zeile der Matrix ein Aminosäurepaar als einen Merkmalsvektor codiert, der eine Paarung einer Aminosäure von der Referenz-Bindersequenz und einer Aminosäure von der Zielbindersequenz beschreibt.

9. Computerimplementiertes Verfahren nach einem vorstehenden Anspruch, wobei der Referenz-Datenspeicher weiter Referenz-Binderzielpaare umfasst, die eine zugeordnete Anzeige von Bindung oder Nichtbindung aufweisen, und wobei das Modell maschinellen Lernens oder das statistische Modell trainiert wird durch:
Zuordnen von jedem Referenz-Binderzielpaar, das einer Anzeige von Bindung oder Nichtbindung zugeordnet ist, zu einem angenommenen zensierten IC₅₀-Wert.

10. Computerimplementiertes Verfahren nach Anspruch 9, weiter umfassend:
Berechnen für jedes Referenz-Binderzielpaar, das einem angenommen zensierten IC₅₀-Wert zugeordnet ist, eines Beitrags zu Bindung durch Integrieren einer zugeordneten statistischen Verteilung über einen Satz von möglichen Bindungsaffinitätswerten.

11. Computerimplementiertes Verfahren nach einem vorstehenden Anspruch, weiter umfassend Ausgeben eines Satzes von Parametern, die dem Modell zugeordnet sind, sodass der Benutzer interpretieren kann, ob das Modell bekannte Moleküle und einen Bindungsaffinitätswert für die bekannten Moleküle angemessen verwendet.

12. Computerimplementiertes Verfahren nach einem vorstehenden Anspruch, wobei das Abfrage-Bindermolekül ein Peptid ist und/oder wobei die zweite Aminosäuresequenz eine MHC-Proteinsequenz oder eine HLA-Proteinsequenz ist.

13. Verfahren zum Erzeugen von mindestens einem kandidatenproteinbindenden Peptid, wobei das Verfahren Folgendes umfasst:
Erhalten einer Aminosäuresequenz einer Vielzahl von Peptiden und einer Aminosäuresequenz eines Proteins;
Bestimmen für jedes Peptid einer vorhergesagten Bindungsaffinität zu dem Protein durch ein Verfahren nach einem der Ansprüche 1 bis 12; und
Auswählen von einem oder mehreren Kandidatenpeptiden der Vielzahl von Peptiden basierend auf der jeweiligen vorhergesagten Bindungsaffinität.

14. Bindungsaffinitäts-Vorhersagesystem zur Vorhersage einer Bindungsaffinität eines Abfrage-Bindermoleküls an ein Abfrage-Zielmolekül, wobei das Abfrage-Bindermolekül eine erste Aminosäuresequenz aufweist und das Abfrage-Zielmolekül eine zweite Aminosäuresequenz aufweist, wobei das System mindestens einen Prozessor in Kommunikation mit mindestens einer Speichervorrichtung umfasst, wobei die mindestens eine Speichervorrichtung darauf gespeichert Anweisungen aufweist, um zu bewirken, dass der mindestens eine Prozessor ein Verfahren nach einem der Ansprüche 1 bis 12 durchführt.

15. Computerlesbares Medium, das Anweisungen umfasst, die, wenn sie von einem Prozessor ausgeführt werden, bewirken, dass der Prozessor ein Verfahren nach einem der Ansprüche 1 bis 12 durchführt.

## Revendications

1. Procédé mis en œuvre par ordinateur de prédiction d'une valeur d'affinité de liaison d'une molécule requête lieur à une molécule requête cible, la molécule requête lieur présentant une première séquence d'acides aminés et la molécule requête cible présentant une deuxième séquence d'acides aminés, le procédé comprenant :
le codage (303) conjoint des première et deuxième séquences d'acides aminés sous forme d'une pluralité d'éléments de données pour générer une paire codée d'acides aminés, chaque élément de données de la paire codée représentant quels acides aminés des première et deuxième séquences d'acides aminés sont appariés au niveau d'un point de contact respectif entre la première séquence d'acides aminés et la deuxième séquence d'acides aminés pour former une paire au point de contact, dans lequel une paire au point de contact est un appariement d'acides aminés provenant d'une molécule lieur et d'une molécule cible qui sont proximaux l'un par rapport à l'autre pour influencer la liaison ; et,
l'application (304) d'un modèle entraîné d'apprentissage automatique ou statistique à la paire codée d'acides aminés pour prédire une valeur d'affinité de liaison, dans lequel le modèle d'apprentissage automatique ou le modèle statistique est entraîné par :
accès, avec au moins un processeur, à un magasin de données de référence de paires de référence lieur-cible comprenant des séquences de lieur de référence et des séquences de cible de référence respectives appariées, chaque paire de référence lieur-cible présentant une valeur de liaison mesurée associée ; et,
codage de chaque paire de référence lieur-cible sous forme d'une pluralité d'éléments de données, chaque élément de données de la paire de référence lieur-cible codée représentant quels acides aminés provenant des séquences de lieur de référence et des séquences de cible de référence respectives appariées sont appariés à un point de contact respectif pour former une paire au point de contact,
dans lequel le modèle est entraîné par estimation d'un ensemble de coefficients qui correspondent aux paires de référence lieur-cible codées et les valeurs d'affinité de liaison mesurées associées respectives ; et,
dans lequel l'application d'un modèle d'apprentissage automatique ou d'un modèle statistique entraîné comprend la récupération de l'ensemble de coefficients de modèle auprès d'un magasin de données, et l'application d'un modèle d'apprentissage automatique ou d'un modèle statistique entraîné comprend une combinaison linéaire des coefficients récupérés et de la paire codée d'acides aminés,
de telle sorte que la valeur d'affinité de liaison prédite est représentative d'une contribution à la liaison de chaque paire au point de contact de la molécule requête lieur et de la molécule requête cible.

2. Procédé mis en œuvre par ordinateur selon la revendication 1, dans lequel la paire codée d'acides aminés est codée sous forme d'un vecteur d'éléments de données.

3. Procédé mis en œuvre par ordinateur selon la revendication 1 ou la revendication 2, dans lequel chaque élément de données est une valeur indiquant la présence d'un appariement d'acides aminés à chaque point de contact.

4. Procédé mis en œuvre par ordinateur selon une quelconque revendication précédente, dans lequel la valeur de liaison mesurée associée est censurée.

5. Procédé mis en œuvre par ordinateur selon une quelconque revendication précédente, comprenant en outre la sortie d'une estimation de probabilité d'exactitude de la valeur d'affinité de liaison prédite.

6. Procédé mis en œuvre par ordinateur selon une quelconque revendication précédente, dans lequel les coefficients sont dérivés par application d'un algorithme d'estimation bayésienne à la paire de référence lieur-cible codée et à la valeur de liaison mesurée associée.

7. Procédé mis en œuvre par ordinateur selon une quelconque revendication précédente, dans lequel chaque paire de référence lieur-cible est codée sous forme d'une matrice creuse, dans lequel chaque ligne représente une paire de référence lieur-cible et dans lequel chaque ligne est associée à une valeur de liaison mesurée.

8. Procédé mis en œuvre par ordinateur selon la revendication 7, dans lequel chaque ligne de la matrice comprend une série de bits, chaque bit correspondant à un appariement possible d'acides aminés pour chaque point de contact et indiquant les acides aminés spécifiques présents dans la paire au point de contact et dans lequel une partition d'une ligne de la matrice code une paire d'acides aminés en tant que vecteur de caractéristiques qui décrit un appariement d'un acide aminé provenant de la séquence de lieur de référence et d'un acide aminé provenant de la séquence de lieur cible.

9. Procédé mis en œuvre par ordinateur selon une quelconque revendication précédente, dans lequel le magasin de données de référence comprend en outre des paires de référence lieur-cible présentant une indication associée de liaison ou de non-liaison et dans lequel le modèle d'apprentissage automatique ou le modèle statistique est entraîné par :
association de chaque paire de référence lieur-cible associée à une indication de liaison ou de non-liaison à une valeur IC₅₀ censurée supposée.

10. Procédé mis en œuvre par ordinateur selon la revendication 9, comprenant en outre :
le calcul, pour chaque paire de référence lieur-cible associée à une valeur IC₅₀ censurée supposée, d'une contribution à la liaison par intégration d'une distribution statistique associée sur un ensemble de valeurs d'affinité de liaison possibles.

11. Procédé mis en œuvre par ordinateur selon une quelconque revendication précédente, comprenant en outre la sortie d'un ensemble de paramètres associés au modèle de telle sorte qu'un utilisateur peut interpréter si le modèle est approprié en utilisant des molécules connues et une valeur d'affinité de liaison pour les molécules connues.

12. Procédé mis en œuvre par ordinateur selon une quelconque revendication précédente, dans lequel la molécule requête lieur est un peptide et/ou dans lequel la deuxième séquence d'acides aminés est une séquence de protéine MHC ou une séquence de protéine HLA.

13. Procédé de génération d'au moins un peptide candidat de liaison à une protéine, le procédé comprenant :
l'obtention de séquences d'acides aminés d'une pluralité de peptides et d'une séquence d'acides aminés d'une protéine ;
la détermination, pour chaque peptide, d'une affinité de liaison prédite à la protéine, par un procédé selon l'une quelconque des revendications 1 à 12 ; et
la sélection d'un ou plusieurs peptides candidats de la pluralité de peptides sur la base de l'affinité de liaison prédite respective.

14. Système de prédiction d'affinité de liaison pour prédire une affinité de liaison d'une molécule requête lieur à une molécule requête cible, la molécule requête lieur présentant une première séquence d'acides aminés et la molécule requête cible présentant une deuxième séquence d'acides aminés, le système comprenant au moins un processeur en communication avec au moins un dispositif de mémoire, ledit au moins un dispositif de mémoire présentant, stockées sur celui-ci, des instructions destinées à amener ledit au moins un processeur à effectuer un procédé selon l'une quelconque des revendications 1 à 12.

15. Support lisible par ordinateur comprenant des instructions qui, lorsqu'elles sont exécutées par un processeur, amènent le processeur à effectuer un procédé selon l'une quelconque des revendications 1 à 12.
